# EUROPEAN PATENT SPECIFICATION

(11) **EP 0 715 523 B1**
(45) Date of publication and mention of the grant of the patent: **18.12.2002**
(21) Application number: 95902384.7
(22) Date of filing: 14.09.1994
(51) Int. Cl.: A61K 48/00, A61K 38/45, A61K 38/46, A61K 47/42, C12Q 1/48, C12N 9/10, C12N 15/54

(54) **PURINE NUCLEOSIDE PHOSPHORYLASE GENE THERAPY FOR HUMAN MALIGNANCY**
PURIN-NUKLEOSID PHOSPHORYLASE GENTHERAPIE DER MENSCHLICHEN BÖSARTIGEN KRANKHEITEN
THERAPIE GENIQUE S'APPLIQUANT AUX TUMEURS MALIGNES CHEZ L'HOMME ET UTILISANT LA NUCLEOSIDE-PHOSPHORYLASE PURIQUE

(30) Priority: 14.09.1993 US 122321
(43) Date of publication of application: 12.06.1996
(73) Proprietor: THE UAB RESEARCH FOUNDATION, Birmingham, AL 35294-0111 (US); Southern Research Institute, Birmingham Alabama 35205 (US)
(72) Inventor: SORSCHER, Eric, J., Birmingham, AL 35205 (US); PARKER, William, B., Birmingham, AL 35202 (US); BENNETT, Leonard, L., Jr., Birmingham, AL 35213 (US)
(74) Representative: Wilkinson, Stephen John
(86) International application number: PCT/US94/10130
(87) International publication number: WO 95/007718

(56) References cited:
- EP-A- 0 392 745
- EP-A- 0 415 731
- PROCEEDINGS OF THE NATIONAL ACADEMY OF SCIENCES,USA, vol.89, January 1992, WASHINGTON D.C.,USA pages 33 - 37 MULLEN ET AL 'TRANSFER OF THE BACTERIAL GENE FOR CYTOSINE DEAMINASE TO MAMMALIAN CELLS CONFERS LETHAL SENSITIVITY TO 5-FLUOROCYTOSINE:A NEGATIVE SELECTION SYSTEM'
- ANTIMICROBIAL AGENTS AND CHEMOTHERAPY, vol.35, no.5, May 1991, WASHINGTON D.C.,USA pages 851 - 857 AVERETT ET AL '6-METHOXYPURINE ARABINOSIDE AS A SELECTIVE AND POTENT INHIBITOR OF VARICELLA-ZOSTER VIRUS'
- GENE THERAPY, vol.1, no.4, July 1994, BASINGSTOKE,UK pages 233 - 238 SORSCHER ET AL 'TUMOR CELL BYSTANDER KILLING IN COLONIC CARCINOMA UTILIZING THE ESCHERICHIA COLI DEOD GENE TO GENERATE TOXIC PURINES'
- PROCEEDINGS OF THE 85TH ANNUAL MEETING OF THE AMERICAN ASSOCIATION FOR CANCER RESEARCH,APRIL 10-13,1994, vol.35, March 1994, SAN FRANCISICO,CA,USA page 374 PARKER ET AL 'TUMOR CELL BYSTANDER KILLING IN COLONIC CARCINOMA UTILIZING THE E.COLI DEOD GENE PRODUCT TO GENERATE TOXIC PURINES'

### SEQUENCE LISTING

(1) GENERAL INFORMATION:
   (i) APPLICANTS: The UAB Research Foundation Southern Research Institute
   (ii) TITLE OF INVENTION: PURINE NUCLEOSIDE PHOSPHORYLASE GENE THERAPY FOR HUMAN MALIGNANCY
   (iii) NUMBER OF SEQUENCES: 4
   (iv) CORRESPONDENCE ADDRESS:
      (A) ADDRESSEE: Patrea L. Pabst
      (B) STREET: 1100 Peachtree Street, Suite 2800
      (C) CITY: Atlanta
      (D) STATE: Georgia
      (E) COUNTRY: USA
      (F) ZIP: 30309-4530
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patentin Release #1.0, Version #1.25
   (vi) CURRENT APPLICATION DATA:
      (A) APPLICATION NUMBER: US
      (B) FILING DATE:
      (C) CLASSIFICATION:
   (viii) ATTORNEY/AGENT INFORMATION:
      (A) NAME: Pabst, Patrea L.
      (B) REGISTRATION NUMBER: 31,284
      (C) REFERENCE/DOCKET NUMBER: IGI102CIP
   (ix) TELECOMMUNICATION INFORMATION:
      (A) TELEPHONE: (404)-815-6508
      (B) TELEFAX: (404)-815-6555
(2) INFORMATION FOR SEQ ID NO:1:
   (i) SEQUENCE CHARACTERISTICS:
      (A) LENGTH: 37 base pairs
      (B) TYPE: nucleic acid
      (C) STRANDEDNESS: single
      (D) TOPOLOGY: linear
   (ii) MOLECULE TYPE: Other nucleic acid
   (xi) SEQUENCE DESCRIPTION: SEQ ID NO:1:
   GATCGCGGCC GCATGGCTAC CCCACACATT AATGCAG 37
(2) INFORMATION FOR SEQ ID NO:2:
   (i) SEQUENCE CHARACTERISTICS:
      (A) LENGTH: 45 base pairs
      (B) TYPE: nucleic acid
      (C) STRANDEDNESS: single
      (D) TOPOLOGY: linear
   (ii) MOLECULE TYPE: Other nucleic acid
   (xi) SEQUENCE DESCRIPTION: SEQ ID NO:2:
   GTACGCGGCC GCTTACTCTT TATCGCCCAG CAGAACGGAT TCCAG 45
(2) INFORMATION FOR SEQ ID NO:3:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (ii) MOLECULE TYPE: Other nucleic acid
   (xi) SEQUENCE DESCRIPTION: SEQ ID NO:3:
   GATCGCTAGC GGGCTCTGAA GACAATCTCT CTCTGC 36
(2) INFORMATION FOR SEQ ID NO:4:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: Other nucleic acid
   (xi) SEQUENCE DESCRIPTION: SEQ ID NO:4:
   GATCGCTAGC TCTTCCTCTA GTCCTCACAA GGTCT 35

## Description

This invention is in the field of cancer therapy, and, in particular, relates to compositions and methods to specifically kill tumor cells by the production of toxic compounds in the tumor cells.

### BACKGROUND OF THE INVENTION

Inefficiency of gene delivery, together with inadequate bystander killing represent two major conceptual hurdles in the development of a toxin-mediated gene therapy for human malignancy.

Gene transfer is rapidly becoming a useful adjunct in the development of new therapies for human malignancy. Tumor cell expression of histocompatibility antigens, cytokines, or growth factors (for example, IL-2, IL-4, GMCSF) appears to enhance immune-mediated clearance of malignant cells in animal models, and expression of chemo-protectant gene products, such as p-glycoprotein in autologous bone marrow cells, is under study as a means of minimizing marrow toxicity following administration of otherwise lethal doses of chemotherapeutic agents *W.F.* Anderson, *Science,* 256:808-813 (1992); A.D. Miller, Nature, 357:455-460 (1992); T. Friedmann, *Science,* 244:1275-1281 (1989); L. Thompson, *Science,* 358:744-746 (1992); R.I. Tepper, et al., *Cell,* 57:503-513 (1989); G.J. Nabel, A. et al., *Hum.* Gene *Therapy* 3:399-410 (1992); B.P. Sorrentino, et al., *Science,* 257 (5066);99-103 (1992).

Theoretically, the most direct mechanism for tumor cell killing using gene transfer is the selective expression of cytotoxic gene products within tumor cells. However, no recombinant enzyme or toxin has proven useful in mediating high levels of toxicity in unselected tumor cells. Classical enzymatic toxins such as pseudomonas exotoxin A, diphtheria toxin and ricin are unlikely to be useful in this context, since these enzymes kill only cells in which they are expressed, and no currently available gene transfer vector is capable of gene delivery to a sufficiently high percentage of tumor cells to make use of the above recombinant enzymes. (W.F. Anderson, *Science,* 256:808-813 (1992); A.D. Miller, *Nature,* 357:455-460 (1992); T. Friedmann, *Science,* 244:1275-1281 (1989); L. Thompson, *Science,* 358:744-746 (1992); R.I. Tepper, et al., *Cell,* 57:503-513 (1989); *G.J.* Nabel, A. et al., *Hum. Gene Therapy* 3:399-410 (1992); B.P. Sorrentino, et al., *Science,* 257 (5066);99-103 (1992); L.L. Houston, In: *Immunotoxins,* A.E. Frankel, Ed. (Kluwer Academic Publishers, Boston), pp. 1-7 (1988)); J.D. Robertus, ibid. pp. 11-23; R.J. Collier, ibid. pp. 25-35 (1988); J.R. Murphy, *Current Topics in Microbiology and Immunology,* 118:235-251 (1985); I.H. Maxwell, et al., *Cancer Research,* 46:4660-4664 (1986); K.D. Bagshawe, *J. Cancer,* 60:275-281 (1989)).

Another strategy that has been developed to selectively kill tumor cells involves the delivery and expression of the HSV dThd kinase gene to replicating tumor cells followed by treatment with ganciclovir. (Z. Ram, et al., *Cancer Res.,* 53:83-88 (1993); F.L. Moolten, *Cancer Res*., 46:5276-5281 (1986); F.L. Moolten, and J.M. Well, *J. Natl*. *Cancer Inst.,* 82:297-300 (1990); B.E. Huber, et al., *Proc. Acad, Sci. USA, 88*:8039-8043 91991); Y. Takamiya, et al., *J. Neuroscience Res.,* 33:493-503 (1992); K.W. Culver et al., *Science,* 256:1550-1552 (1992)). Ganciclovir is readily phosphorylated by the HSV dThd kinase, and its phosphorylated metabolites are toxic to the cell. Very little phosphorylation of ganciclovir occurs in normal human cells. Although only those cells expressing the HSV dThd kinase should be sensitive to ganciclovir (since its phosphorylated metabolites do not readily cross cell membranes), *in vitro* and *in vivo* experiments have shown that a greater number of tumor cells are killed by ganciclovir treatment than would be expected based on the percentage of cells containing the HSV dThd kinase gene. This unexpected result has been termed the "bystander effect" or "metabolic cooperation". It is thought that the phosphorylated metabolites of ganciclovir may be passed from one cell to another through gap junctions. (Z. Ram, et al., *Cancer Res*., 53:83-88 (1993); F.L. Moolten, *Cancer Res*., 46:5276-5281 (1986); F.L. Moolten, and J.M. Well, J. *Natl. Cancer Inst.,* 82:297-300 (1990); B.E. Huber, et al., *Proc. Acad, Sci. USA,* 88:8039-8043 91991); Y. Takamiya, et al., *J. Neuroscience Res.,* 33:493-503 (1992); K.W. Culver et al., *Science,* 256:1550-1552 (1992); M.L. Hooper, and J.H. Subak-Sharpe, *Int. Rev. Cytol*., 69:45-104 (1981)). However, even if a nucleoside monophosphate such as ganciclovir monophosphate were released into the medium by cell lysis, the metabolite would not be able to enter neighboring cells and would likely be degraded (inactivated) to the nucleoside by phosphatases.

Although the bystander effect has been observed in initial experiments using HSV dThd kinase, the limitations present in all current gene delivery vehicles mean that a much greater bystander effect than previously noted will be important to successfully treat human tumors using this approach. One of the difficulties with the current bystander toxicity models is that bystander toxicity with metabolites that do not readily cross the cell membrane will not be sufficient to overcome a low efficiency of gene transfer (for example, transfection, transduction, etc.). In the known toxin gene therapy systems, the efficiency of transduction and/or transfection in vivo is generally low.

An existing protocol for treating brain tumors in humans uses retroviral delivery of HSV dThd kinase, followed by ganciclovir administration. In rat models, using HSV dThd in this context, tumor regressions have been observed (Culver, et al., *Science, 256:* 1550-1552 (1992)). The HSV dThd kinase approach has not proven sufficient in humans thus far; this may in part be due to (1) inadequate bystander toxicity with HSV dThd kinase, and (2) cell killing only of dividing cells using HSV dThd kinase with ganciclovir.

Similarly, the usefulness of *E. coli* cytosine deaminase, which converts 5-fluorocytosine to 5-fluorouracil and has recently been reported to provide substantial bystander toxicity. (Huber, B.E., et al., *Ann. N.Y. Acad. Sci*., *716*: 104-114 (1994)).

Prodrug activation by an otherwise non-toxic enzyme (for example, HSV dThd kinase, cytosine deaminase) has advantages over the expression of directly toxic genes, such as ricin, diphtheria toxin, or pseudomonas exotoxin. These advantages include the capability to (1) titrate cell killing, (2) optimize therapeutic index by adjusting either levels of prodrug or of recombinant enzyme expression, and (3) interrupt toxicity by omitting administration of the prodrug. (W.F. Anderson, *Science,* 256:808-813 (1992); A.D. Miller, Nature, 357:455-460 (1992); T. Friedmann, *Science,* 244:1275-1281 (1989); L. Thompson, *Science,* 358:744-746 (1992); R.I. Tepper, P.K. Pattengale, P. Leder, *Cell,* 57:503-513 (1989); G.J. Nabel, A. et al., *Hum. Gene Therapy* 3:399-410 (1992); B.P. Sorrentino, et al., *Science*, 257 (5066);99-103 (1992); L.L. Houston, IN: *Immunotoxins*, A.E. Frankel, Ed. (Kluwer Academic Publishers, Boston), pp. 1-7 (1988); J.D. Robertus, ibid. pp. 11-23; R.J. Collier, ibid. pp. 25-35 (1988); J.R. Murphy, *Current Topics in Microbiology and Immunology,* 118:235-251 (1985); I.H. Maxwell, et al., *Cancer Research,* 46:4660-4664 (1986); K.D. Bagshawe, *J.* Cancer, 60:275-281 (1989); Z. Ram, et al., *Cancer* Res., 53:83-88 (1993); F.L. Moolten, *Cancer Res.,* 46:5276-5281 (1986); F.L. Moolten, and J.M. Well, *J. Natl. Cancer Inst*., 82:297-300 (1990); B.E. Huber, et al., *Proc. Acad. Sci. USA,* 88:8039-8043 91991); Y. Takamiya, et al., *J. Neuroscience Res.,* 33:493-503 (1992); K.W. Culver et al., *Science,* 256:1550-1552 (1992); P.D. Senter, *FASEB J*., 4:188-193 (1990); P.D. Senter et al., *Cancer Research,* 49:5789-5792 (1989); P.D. Senter et al., *Proc. Natl. Acad. Sci. USA,* 85:4842-4846 (1988)). However, like other recombinant toxic genes, gene transfer of HSV dThd kinase followed by treatment with ganciclovir is neither designed to kill bystander cells nor likely to have broad bystander toxicity in vivo.

An additional problem with the use of the HSV dThd kinase or cytosine deaminase to create toxic metabolites in tumor cells is the fact that the agents activated by HSV dThd kinase (ganciclovir, etc.) and cytosine deaminase (5-fluorocytosine) will kill only cells that are synthesizing DNA (Balzarini, et al., *J. Biol. Chem.,* 268:6332-6337 (1993), and Bruce and Meeker, *J. Natl. Cancer* Inst., 38:401-405 (1967)). Even if a considerable number of nontransfected cells are killed, one would not expect to kill the nondividing tumor cells with these agents.

Thus, there exists a need for a toxin gene therapy method that can overcome the problems of inefficient gene delivery, cell replication-dependent killing and low toxin diffusion between cells. The present invention meets this need by providing a system that can produce cytotoxic purine analogs that can freely diffuse across the cell membranes and kill both dividing and nondividing neighboring cells.

### SUMMARY OF THE INVENTION

Methods and compositions for killing replicating or non-replicating, transfected or transduced targeted mammalian cells and bystander cells, comprising the following steps: (a) transfecting or transducing targeted mammalian cells with a nucleic acid encoding a suitable purine analog nucleoside cleavage enzyme which releases a purine analog from the substrate purine analog nucleoside or providing such enzyme directly to the targeted cells; and (b) contacting the targeted cells expressing or provided with the purine analog nucleoside cleavage enzyme with a substrate for the enzyme to produce a toxic purine base analog thereby killing the targeted cells and also bystander cells not expressing or containing the cleavage enzyme. In the present method of killing cells, a non-human purine analog nucleoside phosphorylase can be an *E. coli* purine analog nucleoside phosphorylase (PNP). The methods can utilize a substrate that is a purine analog nucleoside analog, such as the substrate 9-(β-D-2-deoxyerythropentofuranosyl)-6-methylpurine (MeP-dR).

An isolated nucleic acid encoding a non-human or a genetically modified human PNP in mammalian cells is also provided, and also eukaryotic transfer vectors comprising a nucleic acid encoding a non-human or genetically modified human purine analog nucleoside phosphorylase are provided. The vector can be in a host capable of expressing the PNP or other suitable purine analog nucleoside cleavage enzyme.

### BRIEF DESCRIPTION OF THE DRAWINGS

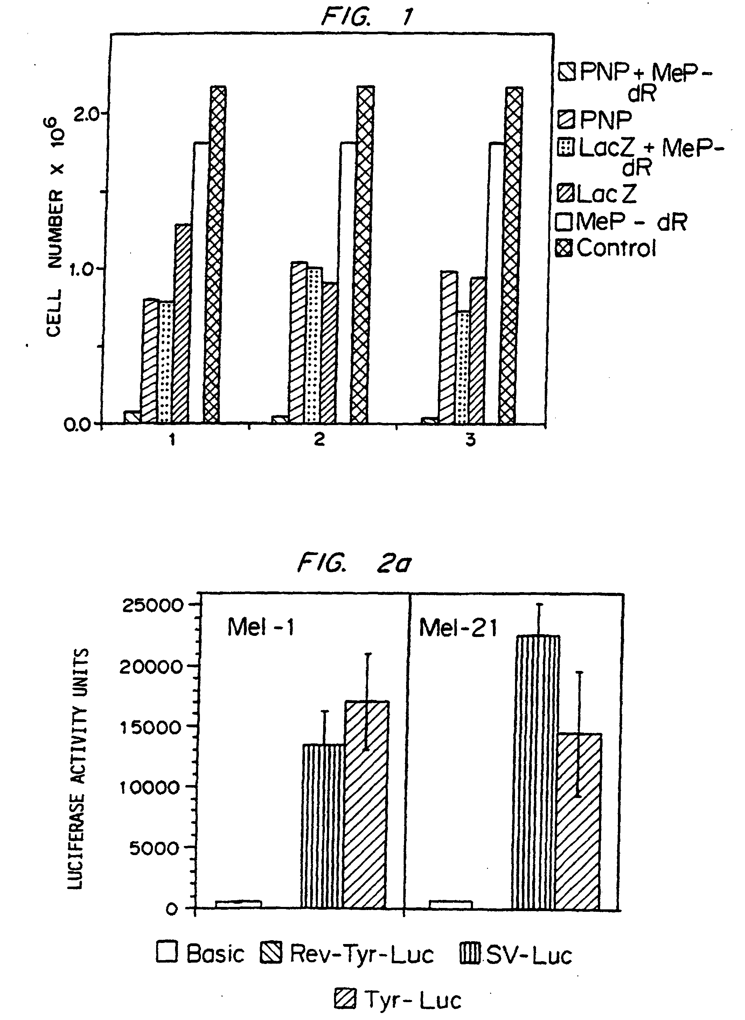

Figure 1 shows the toxicity due to DOTMA-DOPE liposomes used to transfect T84 colon carcinoma cells with 10, 20, or 40 µg (1, 2, and 3 in Fig. 1, respectively) of cDNA containing either the *E. coli* PNP or LacZ genes under the transcriptional control of SV-40 early promoter (SV-PNP and SV-LacZ, respectively), and the additional toxicity when MeP-dR (160 µM) is added to T84 transfected cells expressing the PNP gene (PNP + MeP-dR). Cells transfected with SV-PNP construct were treated with (PNP + MeP-dR) and without (PNP) MeP-dR. Cells transfected with SV-LacZ construct were treated with (LacZ + MeP-dR) and without (LacZ) MeP-dR. Nontransfected cells were treated with (MeP-dR) and without (control) MeP-dR.

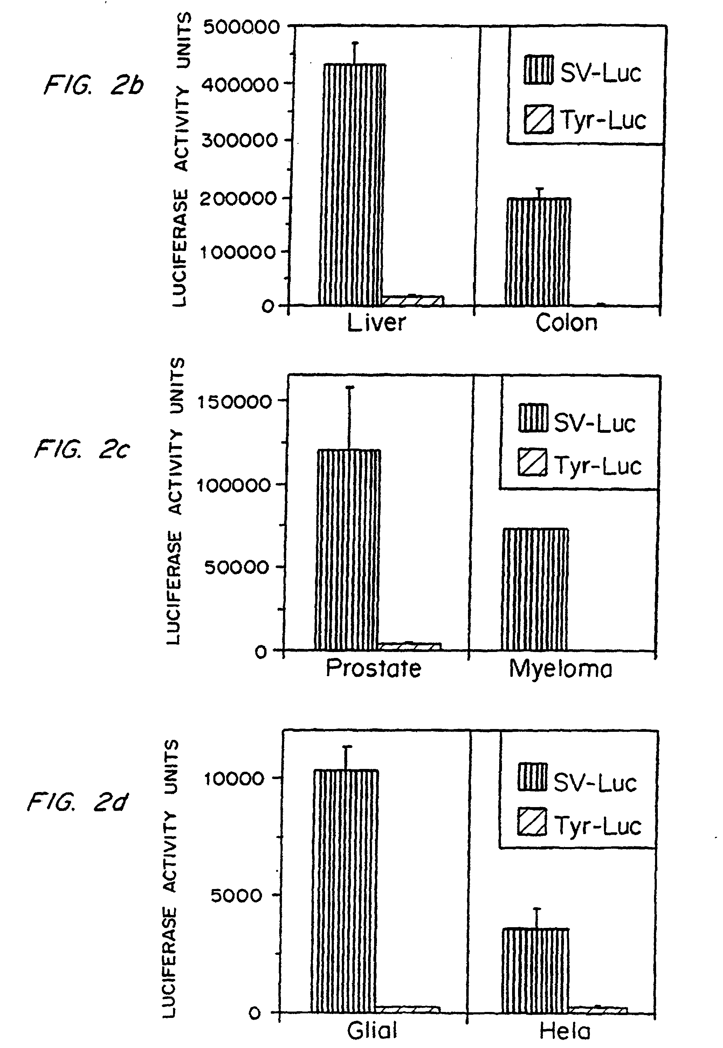

Figures 2a-d show the human tyrosinase transcriptional promoter sequence (Tyr)-restricted expression of the luciferase reporter gene (Luc), to which it was operably linked (Tyr-Luc), in melanoma cells Mel-1 and Mel-21 (Fig. 2a), and the SV40 early promoter (SV) constitutive expression of the luciferase gene (Luc), to which it was operably linked (SV-Luc), in each carcinoma cell line (see Figs. 2a-2d). Rev-Tyr-Luc, Tyr promoter sequence linked to the Luc gene in reverse orientation so that it does not transcribe Luc (no expression). Basic, promoterless Luc gene construct.

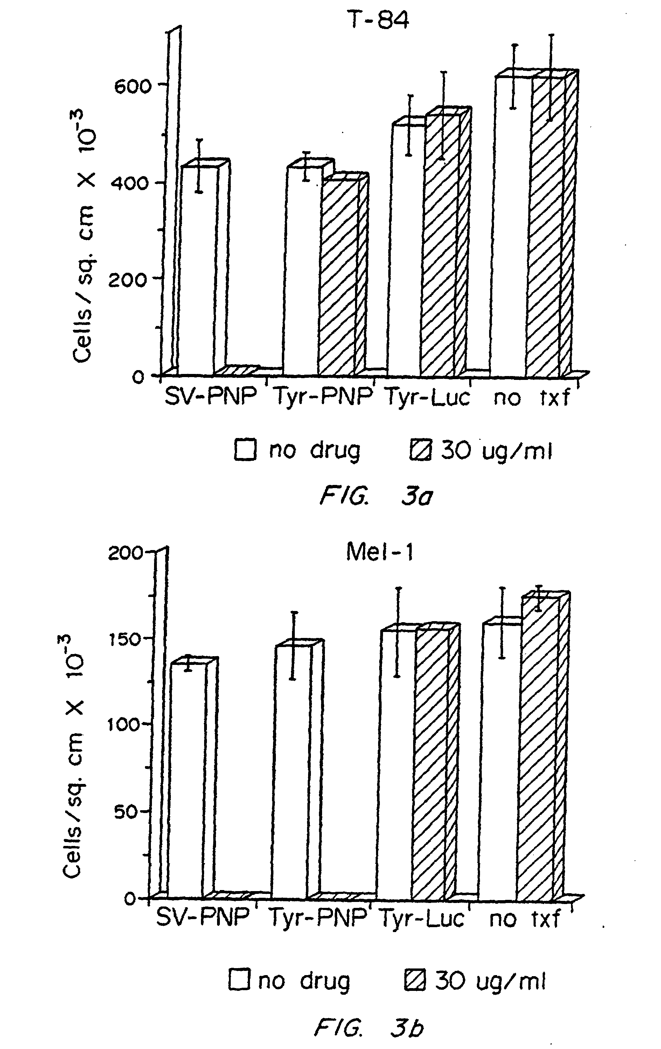

Figures 3a and 3b show the dependence of purine analog nucleoside MeP-dR toxicity on expression of *E. coli* purine analog nucleoside phosphorylase (PNP). SV-PNP, cells transfected with a construct in which the constitutive SV40 early promoter is operably linked to the PNP gene; Tyr-PNP, cells transfected with a construct in which the melanoma specific human tyrosinase promoter sequence is operably linked to PNP gene; Tyr-Luc, cells transfected with a construct in which melanoma specific human tyrosinase promoter sequence is operably linked to luciferase reporter gene; no-txf, cells not transfected with a recombinant construct. T-84, carcinoma cell line (3a); Mel-1, melanoma cell line (3b).

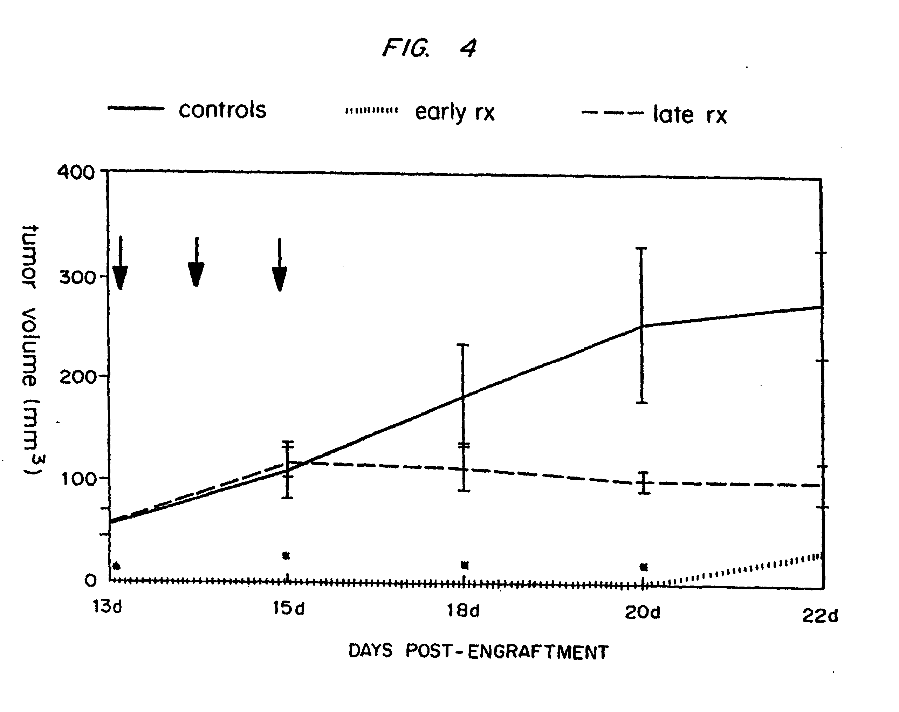

Figure 4 shows the difference in *in vivo* development of tumors in athymic nude mice engrafted with murine mammary carcinoma 16c cells transduced with the recombinant retroviral expression vector LN/PNP (which directs expression of *E. coli* PNP) depending on time of administration of MeP-dR prodrug. No injection of MeP-dR (control); injection of MeP-dR on days 1-4 post engraftment (early rx); injection of MeP-dR on days 13-15 post engraftment (late rx).

### DETAILED DESCRIPTION OF THE INVENTION

The invention provides a method of killing replicating or non-replicating, transfected or transduced mammalian cells and bystander cells, comprising the following steps: (a) transfecting or transducing targeted mammalian cells with a nucleic acid encoding a suitable purine analog nucleoside cleavage enzyme which releases a purine analog from the substrate purine analog nucleoside or providing such enzyme directly to the targeted cells; and (b) contacting the targeted cells expressing or provided with the purine analog nucleoside cleavage enzyme with a substrate for the enzyme to produce a toxic purine base analog thereby killing the targeted cells and also bystander cells not expressing or containing the cleavage enzyme.
Thus, in the presence of substrate, the cleavage enzyme produces a toxic product. It should be appreciated that a "non-human or modified human purine analog nucleoside phosphorylase (PNP)" includes the use of both types of PNP in the same therapeutic regimen for the purine analog nucleoside cleavage enzyme. The killing can occur *in vitro* or *in vivo*, including as human therapy.

Some of the methods and compositions, exemplified below, involve transfecting cells with the *E. coli* DeoD gene (encoding a purine analog nucleoside phosphorylase (PNP)) and subsequently treating with a nontoxic purine nucleoside analog (for example, deoxyadenosine or deoxyguanosine analogs, including N7 analogs), which is converted to a toxic purine analog. *E. coli* PNP differs from human PNP in its more efficient acceptance of adenine and certain guanine-containing nucleoside analogs as substrates. (K.F. Jensen and P. Nygaard, Eur. *J. Biochem.*, 51:253-265 (1975); J. Doskocil and A. Holy, *Collection Czechoslov. Chem.* Commun., 42:370-383 (1977) ; R.E. Parks, et al., (Purine Nucleoside Phosphorylase) IN: *Molecular Actions and Targets for Cancer Chemotherapeutic Agents,* (Academic Press, Inc.), pp. 229-252, 1988; M.S. Hershfield, et al., *Proc. Natl. Acad. Sci. USA,* 88:7185-7189 (1991)). *E. coli* PNP expressed in tumor cells cleaves the nucleoside, liberating a toxic purine analog. Purine analogs freely diffuse across cell membranes, whereas nucleoside monophosphates such as those generated using HSV Thd kinase, generally remain inside the cell in which they are formed. A toxic adenine analog formed after conversion by *E. coll* PNP can be converted by adenine phosphoribosyl transferase to toxic nucleotides and kill all transfected cells, and diffuse out of the cell and kill surrounding cells that were not transfected (bystander cells).

### Enzymes Catalyzing Purine Analog Conversion

### Phosphorylase (PNP) Selection

Two classes of enzymes can be used: phosphorylases and nucleosidases. A PNP useful in the methods and compositions described herein catalyzes the conversion of purine analog nucleosides plus inorganic phosphate to free the toxic purine analog plus ribose-1-phosphate (or deoxyribose-1-phosphate):${\text{purine analog nucleosides + PO}}_{\text{4}} \text{≒ purine analog} {\text{+ ribose-1-PO}}_{\text{4}} \text{(or deoxyribose-1-phosphate).}$

Non-mammalian and modified human or modified other mammalian PNPs can be used. The non-mammalian PNP can be an *E. coli* purine analog nucleoside phosphorylase. However, any PNP which can selectively convert a substrate to produce a toxic purine analog can be utilized. Thus, modifications in the *E. coli* PNP, which do not affect this activity, are within the scope of the class of enzymes suitable for the described methods and compositions, as are human PNP enzyme molecules that have been modified to cleave purine analog nucleoside to release the toxic purine analog moiety. A method is presented below by which any proposed PNP or other purine analog nucleoside cleavage enzyme can be tested in a cell for its ability to convert a given substrate from a relatively nontoxic form to a toxin for the cells. Table 1 lists organisms which possess an enzyme that cleaves adenine-containing nucleosides to adenine and so are useful in the methods described herein. Table 1 also shows that humans and the malaria parasite *Plasmodium falciparum* do not possess an enzyme useful in the described methods. Thus, to be useful in the methods described herein, a human or *P. falciparum* PNP would have to be modified to be capable of cleaving a purine analog nucleoside substrate to liberate the toxic purine analog. Such modifications can be made at the genetic level or protein level. For example, *in vitro* mutagenesis of the gene encoding the human or *P. falciparum* PNP can be used to alter the gene sequence to encode a PNP that will cleave a particular purine analog nucleoside.

As described above, in a preferred embodiment, the PNP used in the present methods can include genetically modified mammalian or non-mammalian PNP, as well as bacterial PNP, capable of reacting with a substrate that the native PNP in the tumor cell, which is targeted to be killed, will not recognize or recognizes very poorly. Thus, the nucleic acids that encode a useful PNP are present in cells in which they are not naturally found, either because they are from a different organism or because they have been modified from their natural state. The key requirement of the nucleic acids encoding the PNP or other purine analog nucleoside cleavage enzyme is that they must encode a functional enzyme that is able to recognize and act upon a substrate that is not well recognized by the native PNP of the cell.

Nucleosidases or hydrolases are another class of enzymes suitable for the methods and compositions described herein. The definition of a purine analog nucleosidase is an enzyme that catalyzes the conversion of purine analog nucleosides plus water to liberate free toxic purine analogs plus ribose (or deoxyribose):${\text{purine analog nucleoside + H}}_{\text{2}} \text{O ≒ purine analog} \text{+ ribose (or deoxyribose).}$

### Transcriptional Regulation of the PNP Encoding sequence

Since a bacterial PNP is encoded on a prokaryotic gene, the expression of the bacterial PNP in mammalian cells will require an eukaryotic transcriptional regulatory sequence linked to the PNP-encoding sequences. The bacterial PNP gene can be expressed under the control of strong constitutive promoter/enhancer elements that are obtained within widely available commercial plasmids (for example, the SV40 early promoter/enhancer (pSVK3, Pharmacia, Piscataway, NJ, cat. no. 27-4511-01), Moloney murine sarcoma virus long terminal repeat (pBPV, Pharmacia, cat. no. 4274390-01), mouse mammary tumor virus long terminal repeat (pMSG, Pharmacia, cat. no. 27-4506-01), and the cytomegalovirus early promoter/enhancer (pCMVβ, Clontech, Palo Alto, CA, cat. no. 6177-1)).

Selected populations of cells can also be targeted for destruction by using genetic transcription regulatory sequences that restrict expression of the bacterial PNP (or other suitable purine analog nucleoside cleavage enzyme) coding sequence to certain cell types, a strategy that is referred to as "transcription targeting". A candidate regulatory sequence for transcription targeting must fulfill two important criteria as established by experimentation: (i) the regulatory sequence must direct enough gene expression to result in the production of enzyme in therapeutic amounts in targeted cells, and (ii) the regulatory sequence must not direct the production of sufficient amounts of enzyme in non-targeted cells to impair the therapeutic approach. In this form of targeting, the regulatory sequences are functionally linked with the PNP sequences to produce a gene that will only be activated in those cells that express the gene from which the regulatory sequences were derived. Regulatory sequences that have been shown to fulfill the criteria for transcription targeting in gene therapy include regulatory sequences from the secretory leucoprotease inhibitor (Garver, Goldsmith et al., *Clin. Res., 41*:308A (1993)), surfactant protein A (Smith et al., Human Gene Therapy, 5: 29-35 (1994), and α-fetoprotein genes (Huber et al., *Proc*. *Natl. Acad. Set. USA, 88*:8039-8043 (1991)). A variation on this strategy is to utilize regulatory sequences that confer "inducibility" so that local administration of the inducer leads to local gene expression. As one example of this strategy, radiation-induced sequences have been described and advocated for gene therapy applications (Weichselbaum, et al., Int. J. Radiation Oncology Biol. Phys., 24:565-567 (1992)). It is expected that bacterial PNP gene expression could be targeted to specific sites by other inducible regulatory elements.

It may be necessary to utilize tissue-specific enhancer/promoters as a means of directing PNP expression, and thereby PNP-mediated toxicity, to specific tissues. For example, human tyrosinase genetic regulatory sequences are sufficient to direct PNP toxicity to malignant melanoma cells. Prior work by Vile and Hart, Cancer Research, 53:962-967 (1993) has shown that mouse tyrosinase sequences from the 5' flanking region (-769 bp from the transcriptional start site) of the gene were capable of directing reporter gene expression to malignant melanoma cells, but not to other cell types. Although the mouse and human tyrosinase sequences in the 5' flanking region are similar, Shibata et al., The Journal of Biological Chemistry, 267:20584-20588 (1992) have published data showing that the human 5' flanking sequences in the same region used by Vile and Hart (-616 bp from the transcriptional start site) did not confer tissue specific expression. Although the report by Shibata, et al., suggested that the 5' flanking region would not be useful to target gene expression to tyrosinase expressing cells (melanomas or melanocytes), a slightly different upstream fragment from that used by Shibata, et al., can in fact direct reporter or bacterial PNP gene expression specifically to melanoma cells, as shown below in Example 2.

As described in Example 2, the 5' flanking region of the human tyrosinase gene was amplified by the polymerase chain reaction from human genomic DNA. The primers were designed to amplify a 529 bp fragment that extended -451 to +78 bp relative to the transcription start site by using a published sequence of the human tyrosinase gene and flanks (Kikuchi, et al., Biochimica et Biophysica Acta, 1009:283-286 (1989)). The fragment was shown by reporter gene assays to be able to direct reporter gene expression in melanoma cells. The same tyrosinase fragment was used to direct PNP expression within a plasmid vector and shown to result in PNP mediated toxicity only in melanoma cells. Therefore, human tyrosinase sequences are useful to direct PNP expression to human melanoma cells. These same sequences could be useful to direct other therapeutic gene expression in melanoma cells or melanocytes. Other tissue-specific genetic regulatory sequences and elements can be used to direct expression of a gene encoding a suitable purine analog nucleoside cleavage enzyme to specific cell types other than melanomas.

**TABLE 1**

| **Organisms which can cleave adenine-containing nucleosides to adenine.** | |
|---|---|
| Organism | Enzyme |
| Leishmania Donvani | Hydrolase (Mostly dAdo) |
| Trichomonas vaginalis | Phosphorylase (Does not contain ARPTase) |
| Trypanosoma Cruzi | Hydrolase |
| Schistosoma Mansoni | Phosphorylase |
| Leishmania Tropica | Hydrolase |
| Crithidia Fasciculata | Hydrolase |
| Aspergillus and Penicillium (fungi) | Hydrolase |
| Erwinia carotovora | Phosphorylase |
| Helix pomatia (snail) | Phosphorylase |
| Ophiodon Eloogatus (lingcod) | Phosphorylase |
| E. coli | Phosphorylase |
| Salmonella typhimurium | Phosphorylase |
| Bacillus subtilis | Phosphorylase |
| Clostridium | Phosphorylase |
| mycoplasmas | Phosphorylase |

| **Organisms that cannot (or poorly) convert adenine nucleosides to adenine.** | |
|---|---|
| Organism | Enzyme |
| Human | Phosphorylase |
| P. falciparum | Phosphorylase |

### Substrate Selection

A purine analog nucleoside which is a substrate for the enzyme to produce a toxic substance which kills the cells is referred to herein as a "prodrug". Any deoxypurine analog nucleoside composed of the bases listed below and in Table 2, should be a substrate for the *E. coli* PNP or other equivalent purine analog nucleoside cleavage enzyme. A requisite is that the analog must have a low toxicity at the nucleoside level (that is, as a prodrug). Using ribose- or deoxyribose-containing substrates, *E. coli* PNP can selectively produce a variety of toxic guanine analogs, such as 6-thioguanine or 3-deazaguanine, that are attached to ribose or deoxyribose via the N-7 position in the guanine ring (for example, D.G. Streeter, et al., *Biochemical Pharmacology, 29*:1791-1797 (1979)). The strategy described here for therapeutic PNP gene transfer implicates new uses for several broad classes of specifically activatable cytotoxic purine analogs in the treatment of human malignancy. Because the growth fraction is very small in most tumors, it is preferable to select compounds that are active against both dividing and nondividing cells. It appears that some of the toxic purine analogs produced using *E. coli* PNP in the present method are toxic to nondividing as well as dividing cells. Specific examples of suitable purine analog nucleosides that will work in the compositions and methods described herein can be tested according to the protocols set forth in the Examples.

In a preferred embodiment described in the Examples, the substrate is 9-(β-D-2-deoxyerythropentofuranosyl)-6-methylpurine (MeP-dR). Although MeP-dR is relatively non-toxic, the therapeutic index of this compound can be enhanced. For instance, if the toxicity of MeP-dR is due to phosphorylation by a deoxynucleoside kinase, then analogs that cannot be phosphorylated, such as 5'-deoxy-MeP-dR, can be synthesized and used as the prodrug to generate MeP *in vivo*.

The compounds 6-methylpurine-2'-deoxyriboside (Gene Therapy, 1: 233-238, 1994),
2-amino-6-chloro-1-deazapurine riboside (*Biochem. Pharmacol*., *33:* 261-271, 1984), and
7-ribosyl-3-deazaguanine (*Biochem. Pharmacol*., *29:* 1791-1787, 1979) are examples of prodrugs that are useful substrates for the *E. coli* PNP. They are much less toxic than their respective purine analogs and are very good substrates for *E. coil* PNP and poor substrates for mammalian PNP.

*E. coli* PNP can also cleave arabinofuranosyl 2-fluoroadenine (F-araA) or 2-fluoro-2'-deoxyadenosine (F-dAdo) to 2-fluoroadenine (F-Ade), a very toxic compound. F-araA and F-dAdo have demonstrated *in vivo* anti-tumor activity. F-araA is approved for use in people for the treatment of leukemias. Because the anti-tumor activity of these compounds is not related to the production of F-Ade, it is expected that treatment of a tumor that expresses *E. coli* PNP or other suitable purine analog nucleoside cleavage enzyme with F-araA or F-dAdo would lead to an enhanced antitumor effect due to the cell killing from the selective production of F-Ade in the tumor cells.

As shown in Example 3, further studies, using the methods described herein, can identify other purine analog nucleoside analogues with a therapeutic index superior even to that shown here for MeP-dR. Expression of *E. coli* PNP (or other suitable purine analog nucleoside cleavage enzyme) within malignant cells can be used to screen a variety of substrates to determine which produce toxic purine analogs, such as 2-fluoroadenine (substrate: 2-fluoro-2'-deoxyadenosine with or without 2'-deoxycytidine added to ameliorate toxicity), or 2-azaadenine, 4-amino-pyrazolo [3,4-d] pyrimidine.

### Gene Delivery

A PNP gene can be delivered to mammalian cells by a variety of methods well known to those skilled in the art. Viral vectors are used in a first preferred embodiment. Examples include retrovirus, adenovirus, adeno-associated virus, herpes virus, and vaccinia virus, among others. The Examples below describe the construction of suitable recombinant viruses and the use of adenovirus for the transfer of bacterial PNP into mammalian cells.

Non-viral gene delivery can also be used. Examples include diffusion of DNA in the absence of any carriers or stabilizers ("naked DNA"), DNA in the presence of pharmacologic stabilizers or carriers ("formulated DNA"), DNA complexed to proteins that facilitate entry into the cell ("molecular conjugates"), or DNA complexed to lipids. The use of lipid-mediated delivery of the bacterial PNP gene to mammalian cells is exemplified below. More particularly, cationic liposome-mediated transfer of a plasmid containing a non-human PNP gene is demonstrated. However, other gene transfer methods will also generally be applicable because the particular method for transferring the PNP gene to a cell is not determinative of successful tumor cell killing. Thus, gene transduction, utilizing a virus-derived transfer vector, further described below, can also be used. Such methods are well known and readily adaptable for use in the gene-mediated toxin therapies described herein. Further, these methods can be used to target certain diseases and cell populations by using the targeting characteristics of a particular carrier of the gene encoding a suitable purine analog nucleoside cleavage enzyme, such as *E. coli* PNP.

Apathogenic anaerobic bacteria have been used to selectively deliver foreign genes into tumor cells. For example, *Clostridium acetobutylicum* spores injected intravenously into mice bearing tumors, germinated only in the necrotic areas of tumors that had low oxygen tension (Lemmon, M.J., et al., *Proc. Am. Ass. for Cancer Research*, 35: 374 (abstract no. 2231) (1994)). Using the standard PNP assay described below (Example 1), *Clostridium perfringens* (sigma Chemical Co., St. Louis, MO) was found to exhibit enzyme activity capable of converting MeP-dR to MeP. This finding suggests a mechanism to selectively express bacterial PNP activity in tumor masses with necrotic, anaerobic centers. Thus, tumors can be infected with such strains of *Clostridium* and then exposed to a purine analog such as MeP-dR. The PNP activity of the *Clostridium* bacteria growing in the anaerobic center of the tumor tissue should then convert the MeP-dR to MeP, which then is released locally to kill the tumor cells.

The rapidly advancing field of therapeutic DNA delivery and DNA targeting also includes vehicles such as "stealth" and other antibody-conjugated liposomes (including lipid-mediated drug targeting to colonic carcinoma), receptor-mediated targeting of DNA through cell specific ligands, lymphocyte-directed tumor targeting, and highly specific therapeutic retroviral targeting of murine glioma cells in vivo (S.K. Huang et al., *Cancer Research,* 52:6774-6781 (1992); R.J. Debs et al., *Am. Rev. Respir. Dis.,* 135:731-737 (1987); K. Maruyama, et al., *Proc. Natl. Acad. Sci. USA,* 87:5744--5748 (1990); P. Pinnaduwage, and L. Huang., *Biochemistry,* 31:2850-2855 (1992); A. Gabizon, and D. Papahadjopoulos, *Proc. Natl. Acad. Sci. USA,* 85:6949-6953 (1988); S.A. Rosenberg, et al., *N. England J. Med.,* 323:570-578 (1990); K. Culver et al., Proc. Natl. Acad. Sci. USA. 88:3155-3159 (1991); G.Y. Wu, and C.H. Wu, *J. Biol. Chem. 263,* No. 29:14621-14624 (1988); E. Wagner, et al., *Proc. Natl. Acad. Sci. USA,* 87:3410-3414 (1990); D.T. Curiel et al., *Hum. Gene Ther.,* 3:147-154 (1992); D.C. Litzinger, and L. Huang, *Biochimica et Biophysica Acta,* 1104:179-187 (1992); v.s. Trubetskoy, et al., *Biochimica et Biophysics Acta,* 1131:311-313 (1992)). It is not known whether rates of cell division within a human glioma (where tumor cell division is likely to be substantially less than in experimental brain tumor models) will support adequate levels of retroviral integration to permit tumor regression using HSV dThd kinase. The approach described here, within the context of a gene targeting mechanism either directed toward dividing tumor cells or tumor neovascularization, offers an improved means by which a small subset of tumor cells could be established within a growing tumor mass, which would mediate rapid tumor involution and necrosis after the appropriate signal, that is, after administration of the substrate (prodrug) for a suitable purine analog nucleoside cleavage enzyme, such as *E. coli* PNP present in or adsorbed to tumor cells.

### Methods of Treatment

The method of treatment basically consists of providing to cells the PNP gene and then exposing the cells to an appropriate substrate which is converted to a toxic substance which kills the cells expressing the PNP gene as well as those in the vicinity of the PNP gene expressing cells. The PNP gene can be administered directly to the targeted cells as systemically in combination with a targeting means, such as through the selection of a particular viral vector or delivery formulation. Cells can be *in vivo*, within the patient to be treated, or treated *in vitro,* then injected into the patient. Following introduction of the PNP gene into cells in the patient, prodrug is administered, systemically or locally, in an effective amount to be converted by the PNP into a sufficient amount of toxic substance to kill the targeted cells.

### A. Treatment of Tumors

The *E. coli* PNP gene can also be used as part of a strategy to treat metastatic solid tumors, such as melanoma, pancreatic, liver or colonic carcinoma. No effective therapy for metastatic tumors of these types currently exists. In this method, plasmid DNA containing a PNP gene under the control of tumor specific promoters is used. For example, the tyrosinase promoter is highly specific for mediating expression in melanoma cells, and will not lead to gene expression in other tissue types (Vile and Hart, *Cancer Res.,* 55:962-967 (1993)). The PNP gene under the regulatory control of this promoter, therefore, should be activated predominantly within a melanoma tumor and not elsewhere within a patient (see Example 2 and Figures 2a-d below). Promoters specific for other tumor types, for example, promoters active in the rapidly dividing endothelial cells present in all solid tumors such as the tie gene promoter in neovascular cells, can be used to specifically activate PNP only within a primary or metastatic tumor. In this method, plasmid DNA containing PNP under the control of a tumor specific promoter is delivered to cells using cationic liposomes. For example, based on animal studies, 100-400 mg plasmid DNA complexed to 1200-3600 micromoles of a 1:1 mixture of the lipids DOTMA (1,2,-dioleyloxypropyl-3-trimethyl ammonium bromide) and DOPE (dioleoyl phosphatidylethanolamine) can be used to deliver the PNP gene to tumor metastases in patients. Prodrug in the above described amounts can then be administered as described above.

The PNP gene can be used to activate prodrugs in the treatment of human brain cancer (Recombinant Advisory committee of the National Institutes of Health, Protocol #9206-019, Oldfield, et al., approval date 6-1-92). In this method, a cell line producing retroviral particles, in which the viral particles contain the *E. coli* PNP gene, is injected into a central nervous system (CNS) tumor within a patient. An MRI scanner is used to appropriately inject the retroviral producer cell line to within the tumor mass. Because the retrovirus is only fully active within dividing cells and most of the dividing cells within the cranium of a cancer patient are within the tumor, the retrovirus is primarily active in the tumor itself, rather than in non-malignant cells within the brain. Clinical features of the patient including tumor size and localization, determine the amount of producer cells to be injected. For example, a volume of producer cells in the range of 30 injections of 100 microliters each (total volume 3 ml with approximately 1 x 10⁸ producer cells/ml injected) are given under stereotactic guidance for surgically inaccessible tumors. For tumors which can be approached intraoperatively, 100 µl aliquots are again injected (at about 1 x 10⁸ cells/ml) with total injected volumes up to 10 ml. Using *E. coli* PNP gene transfer, followed by MeP-dR administration, is designed to permit both bystander killing and toxicity to non-dividing cells and is thus designed for tumor involution much greater than previous attempts using HSV dThd and ganciclovir.

The destruction of selected populations of cells can be achieved by targeting the delivery of the bacterial PNP gene or other gene encoding an enzyme capable of cleaving purine analog from a purine analog nucleoside, such as, adenine from adenine-containing nucleosides (see, for example, Table 1), as described above. The natural tropism or physiology of viral vectors can also be exploited as a means of targeting specific cell types. For example, retroviruses are well known to become fully active only in replicating cells. This fact has been used as the basis for selective retroviral-mediated gene transfer to replicating cancer cells growing within a site where the normal (nonmalignant) cells are not replicating in both animal and human clinical studies (Culver et al., 1992; Ram et al., Cancer Gene Ther., 1:1 (1993)). Alternatively, the viral vector can be directly administered to a specific site such as a solid tumor, where the vast majority of the gene transfer will occur relative to surrounding tissues. This concept of selective delivery has been demonstrated in the delivery of genes to tumors in mice by adenovirus vectors (Tang, et al., Cancer Gene Ther., 1: 15-20 (1994). Molecular conjugates can be developed so that the receptor-binding ligand will bind only to selective cell types, as has been demonstrated for the lectin-mediated targeting of lung cancer cells (Batra, et al., 1994).

Thus, cells, whether malignant or not, can be killed by the present method based on the ability to selectively transfer or express a gene encoding a suitable purine analog nucleoside cleavage enzyme, to at least a small percentage of cells.

Recently, for example, it has been shown that intravenous injection of liposomes carrying DNA can mediate targeted expression of genes in certain cell types. Targeting of a gene encoding a purine analog nucleoside cleavage enzyme or expression of the gene to a small fraction of the cells in a tumor mass followed by substrate administration could be adequate to mediate involution (Zhu, et al., *Science, 261*:209-211 (1993)). Through the substantial bystander effect and killing of nondividing cells demonstrated in the Examples, the present method can be used to destroy the tumor.

### B. Treatment of Virally Infected Cells

In addition to killing tumor cells, the methods described herein can also be used to kill virally infected cells. In a virus-killing embodiment, the selected gene transfer method is chosen for its ability to target the expression of the cleavage enzyme in virally infected cells. For example, virally infected cells may utilize special viral gene sequences to regulate and permit gene expression, that is, virus specific promoters. Such sequences are not present in uninfected cells. If the PNP gene is oriented appropriately with regard to such a viral promoter, the cleavage enzyme would only be expressed within virally infected cells, and not other, uninfected, cells. In this case, virally infected cells would be much more susceptible to the administration of MeP-dR or other substrates designed to be converted to toxic form by non-human or modified human PNP.

### C. Administration of Genetically Engineered Cells

For certain applications, cells that receive the PNP gene are selected and administered to a patient. This method most commonly involves ex *vivo* co-transfer of both the gene encoding the cleavage enzyme, such as the bacterial PNP gene, and a second gene encoding a therapeutic protein gene. The cells that receive both genes are reinfused into the host patient where they can produce the therapeutic protein until the prodrug, such as MeP-dR, is administered to eliminate the engineered cells. This method should be useful in "cell therapies", such as those used on non-replicating myoblasts engineered for the production of tyrosine hydroxylase within the brain (Jiao, et al., *Nature, 362*:450 (1993)).

### D. Direct Delivery of the PNP Enzyme to Cells

The bystander killing conferred by the bacterial PNP protein plus prodrug combination can also be achieved by delivering the PNP protein to the target cells, rather than the PNP gene. For example, a PNP enzyme capable of cleaving purine analog nucleosides as described above, is manufactured by available recombinant protein techniques using commercially available reagents. As one example of a method for producing the bacterial PNP protein, the *E. coli PNP* coding sequence is ligated into the multiple cloning site of pGEX-4T-1 (Pharmacia, Piscataway, NJ) so as to be "in frame", with the glutathione-S-transferase (GST) fusion protein using standard techniques (note that the cloning site of this vector allows insertion of coding sequences in all three possible translational reading frames to,facilitate this step). The resulting plasmid contains the GST-PNP fusion coding sequence under transcriptional control of the IPTG-inducible, prokaryotic *tac* promoter. *E. coli* cells are transformed with the recombinant plasmid and the tac promoter induced with IPTG. IPTG-induced cells are lysed, and the GST-PNP fusion protein purified by affinity chromatography on a glutathione Sepharose 4B column. The GST-PNP fusion protein is eluted, and the GST portion of the molecule removed by thrombin cleavage. All of these techniques and reagents are provided in a commercially available kit (Pharmacia, Piscataway, NJ, catalog no. 27-457001). Other methods for recombinant protein production are described in detail in published laboratory manuals (see, for example, Chapter 16, "Protein Expression" from *Current Protocols in Molecular Biology*).

Since the bacterial PNP activates the prodrugs into diffusible toxins, it is only necessary to deliver the PNP protein to the exterior of the target cells prior to prodrug administration. The PNP protein can be delivered to targets by a wide variety of techniques. One example would be the direct application of the protein with or without a carrier to a target tissue by direct application, as might be done by directly injecting a tumor mass within an accessible site. Another example would be the attachment of the PNP protein to a monoclonal antibody that recognizes an antigen on the tumor site. Methods for attaching functional proteins to monoclonal antibodies have been previously described, and detailed protocols are described in published laboratory manuals (for example, Chapter 9, "Labeling Antibodies" from *Antibodies: A Laboratory Manual*). The PNP conjugated monoclonal antibody is systemically administered, for example, intravenously (IV), and attaches specifically to the target tissue. Subsequent systemic administration of the prodrug will result in the local production of diffusible toxin in the vicinity of the tumor site. A number of studies have demonstrated the use of this technology to target specific proteins to tumor tissue (see, for example, Senter, et al., *Bioconjugate Chem*., 2:447-451, 1991; Bagshawe, K.D., *Br. J. Cancer,* 60:275-281, 1989; Bagshawe, et al., *Br. J. Cancer,* 58:700-703, 1988; Senter, et al., Bioconjugate *Chem*., 4:3-9, 1993; Battelli, et al., *Cancer Immunol. Imununother.,* 35:421-425, 1992; Pietersz and McKenzie, *Immunolog. Reviews,* 129:57-80 (1992); and Roffler, et al., Biochem. *Pharmacol,* 42:2062-2065 (1991)). Other ligands, in addition to monoclonal antibodies, can be selected for their specificity for a target cell and tested according to the methods taught herein.

Another example of protein delivery to specific targets is that achieved with liposomes. Methods for producing liposomes are described in published laboratory manuals (for example, *Liposomes: A Practical Approach*). Liposomes can be targeted to specific sites by the inclusion of specific ligands or antibodies in their exterior surface, as has been described in detail by Van Berkel et al., in which specific liver cell populations were targeted by the inclusion of asialofetuin in the liposomal surface (Van Berkel et al., Targeted Diagnosis and Therapy, 5:225-249 (1991)). Specific liposomal formulations can also achieve targeted delivery, as best exemplified by the so-called Stealth™ liposomes that preferentially deliver drugs to implanted tumors (Allen, Liposomes in the Therapy of Infectious Diseases and Cancer, 405-415 (1989)). After the liposomes have been injected or implanted, unbound liposome is allowed to be cleared from the blood, and the patient is treated with the purine analog nucleoside prodrug, such as MeP-dR, which is cleaved to MeP by the *E. coli* PNP or other suitable cleavage enzyme at the targeted site. Again, this procedure requires only the availability of an appropriate targeting vehicle. The following references are examples of the targeting of specific proteins to tumor tissue (Hughes et al., *Cancer Research,* 49: 6214-6220 (1989); and Litzinger and Huang, *Biochimica et Biophysica Acta, 1104*:179-187 (1992)). In a broader sense, the strategy of targeting can be extended to specific delivery of the prodrug following either PNP protein, or gene, delivery.

### E. Administration of Substrates

The formula of Freireich, et al. (*Cancer Chemother. Rep.*, 50:219-244, (1966)) can be used to determine the maximum tolerated dose of substrate for a human subject. For example, based on systemically administered dose response data in mice showing that a dose of 25 mg (MeP-dR) per kg per day for 9 days (9 doses total) resulted in some toxicity, but no lethality, a human dosage of 75 mg MeP-dR/m² was determined according to the formula: 25 mg/kg x 3 = 75 mg/m². This amount or slightly less should result in maximal effectiveness of tumor cell killing in humans without killing the subject. This standard of effectiveness is accepted in the field of cancer therapy. However, more preferred is a range of from about 10% to 1% of the maximum tolerated dosage (for example, 7.5 mg/m² - 0.75 mg/m²) . Furthermore, it is understood that modes of administration that permit the substrate to remain localized at or near the site of the tumor will be effective at lower doses than systemically administered substrates.

The substrate may be administered orally, parenterally (for example, intravenously), by intramuscular injection, by intraperitoneal injection, or transdermally. The exact amount of substrate required will vary from subject to subject, depending on the age, weight and general condition of the subject, the severity of the disease that is being treated, the location and size of the tumor, the particular compound used, its mode of administration, and the like. Thus, it is not possible to specify an exact amount. However, an appropriate amount may be determined by one of ordinary skill in the art using only routine experimentation given the teachings herein. Generally, dosage will preferably be in the range of about 0.5 - 50 mg/m², when considering MeP-dR, for example, or a functional equivalent.

Depending on the intended mode of administration, the substrate can be in pharmaceutical compositions in the form of solid, semi-solid or liquid dosage forms, such as, for example, tablets, suppositories, pills, capsules, powders, liquids, or suspensions, preferably in unit dosage form suitable for single administration of a precise dosage. The compositions will include, as noted above, an effective amount of the selected substrate in combination with a pharmaceutically acceptable carrier and, in addition, may include other medicinal agents, pharmaceutical agents, carriers, or diluents. By "pharmaceutically acceptable" is meant a material that is not biologically or otherwise undesirable, which can be administered to an individual along with the selected substrate without causing significant undesirable biological effects or interacting in a deleterious manner with any of the other components of the pharmaceutical composition in which it is contained.

For solid compositions, conventional nontoxic solid carriers include, for example, pharmaceutical grades of mannitol, lactose, starch, magnesium stearate, sodium saccharin, talc, cellulose, glucose, sucrose and magnesium carbonate. Liquid pharmaceutically administrable compositions can, for example, be prepared by dissolving or dispersing, an active compound with optimal pharmaceutical adjuvants in an excipient, such as water, saline, aqueous dextrose, glycerol, or ethanol, to thereby form a solution or suspension. If desired, the pharmaceutical composition to be administered may also contain minor amounts of nontoxic auxiliary substances such as wetting or emulsifying agents, pH buffering agents, for example, sodium acetate or triethanolamine oleate. Actual methods of preparing such dosage forms are known, or will be apparent, to those skilled in this art; for example, see Remington's Pharmaceutical Sciences (Martin, E.W. (ed.) latest edition Mack Publishing Co., Easton, PA.).

For oral administration, fine powders or granules may contain diluting, dispersing, and/or surface active agents, and may be presented in water or in a syrup, in capsules or sachets in the dry state, or in a nonaqueous solution or suspension wherein suspending agents may be included, in tablets wherein binders and lubricants may be included, or in a suspension in water or a syrup. Where desirable or necessary, flavoring, preserving, suspending, thickening, or emulsifying agents may be included. Tablets and granules are preferred oral administration forms, and these may be coated.

Parenteral administration is generally by injection. Injectables can be prepared in conventional forms, either liquid solutions or suspensions, solid forms suitable for solution or suspension in liquid prior to injection, or as emulsions.

The following examples are intended to illustrate certain aspects of the compositions and methods described herein. While the protocols described are typical of those that might be used, other procedures known to those skilled in the art may be alternatively employed.

### EXAMPLES

### Example 1: Effect of MeP-dR on Human Colon Carcinoma

### Cells Expressing E. coli PNP

### Substrate

MeP-dR was chosen because it is 20-fold less toxic than 6-methylpurine (MeP) to HEp-2 cells and it has been used to detect cultures infected with mycoplasma, because mycoplasma express an enzyme similar in function to *E. coll* PNP (J.A. Montgomery and K. Hewson, *J. Med. Chem*., *11*:48-52 (1968). G.J. McGarrity and D.A. Carson, *Experimental Cell Research, 139*:199-206 (1982); M. Hatanka, et al., *In vitro*, *13*:429-433 (1977)).

### Cell lines

T84 colon carcinoma cells (E.J. Sorscher, et al., *Amer. J. Physiol., 262*:C136-C147 (1992)) were grown in Dulbecco's modified Eagle medium containing F12 nutrient medium (DMEM/F12) (GIBCO/BRL, Gaithersburg, MD) in 6 well trays to a density of approximately 1-2 x 10⁵ cells/well (^{~}20% confluency).

### Toxicity of Mep-dR Within Colon Carcinoma Cells

Untransfected T84 colon carcinoma cells were treated with increasing concentrations of either MePdR or MeP. After 5 days the cells were removed from each well and the number of dye excluding cells were determined with the aid of a hemacytometer. Cells were studied both at passage 48 (p. 48) and passage 61 (p.61). MeP was obtained from Sigma Chemical Company, (St. Louis, MO). MeP-dR was synthesized by standard methods as described (J.A. Montgomery, and K. Hewson, *J. Med. Chem*., *11*:48-52 (1968)). The nucleoside and base were dissolved in serum free DMEM/F12 at a concentration of 1 mg/ml and added directly to 1 ml DMEM/F12 with 10% fetal bovine serum at the concentrations described below in order to cover 1-2 x 10⁵ cells/well.

Initial cytopathic effects due to MeP were observed within 24 hours (for example, rounding of cells, with some cells detaching from plate). Viable cells were counted 5 days following addition of drug. The higher concentrations (3.75 µM - 75 µM) of MeP resulted in cell lysis and complete loss of cellular architecture, leaving only cellular debris within wells by day 2 following treatment; trypan blue exclusion was used to confirm viability in cells retaining recognizable structure at all concentrations studied. At lower concentrations MeP-dR did not cause any appreciable cell death and higher concentrations (200 and 400 µM) less than half of the cells were killed. If the toxicity of MeP-dR is due to very low levels of liberation of MeP by human PNP, then combination with selective inhibitors of human PNP could prevent this toxicity (J.A. Montgomery, et al. *J. Medicinal Chem.* 36:55-69 (1993)).

### Enhanced Toxicity of MeP-dR Mediated by E. coli PNP Expression Vectors

A bacterial PNP-encoding sequence was inserted into plasmid expression vector. *E. coli* (strain JM101) chromosomal DNA template was obtained using the method described in N.J. Gay, *J. Bacterial.* 158:820-825 (1984). Two PCR primers GATCGCGGCCGCATGGCTACCCCACACATTAATGCAG (SEQ ID No:1) and GTACGCGGCCGCTTACTCTTTATCGCCCAGCAGAACGGATTCCAG (SEQ ID NO:2) were used to define the full length coding sequence of the *E. coli* DeoD gene (5) and to incorporate Notl sites at both 5' and 3' ends of the desired product. After 30 cycles of amplification (94°C x 1 min denaturation, 50°C x 2 min annealing, and 72°C x 3 min elongation using 1 ng template, 100 ng of each primer in a 100 µl reaction mixture containing 2.5 units taq polymerase, 200 µm each dNTP, 50 mM KCl, 10 mM Tris Cl (pH 8.3), 1.5 mM MgCl₂ and 0.01% gelatin (weight/vol), a single PCR product of the predicted size (716 base pairs) was obtained. This product was extracted with phenol/chloroform, precipitated with ethanol, digested with NotI, and gel purified using the Gene Clean kit, (Bio. 101, La Jolla, CA).

The amplified bacterial PNP sequence was added to a plasmid eukaryotic expression vector. In order to obtain a vector capable of directing eukaryotic expression of *E. coli* PNP, the LacZ gene was excised from pSVβ (Clontech, Palo Alto, CA) by digestion with NotI, the vector backbone was dephosphorylated (calf intestinal alkaline phosphatase, GIBCO BRL, Gaithersburg, MD) and gel purified as above. The PNP insert, prepared as above, was then ligated into the NotI ends of the plasmid backbone in order to create a new construct with PNP expression controlled by the SV-40 early promoter. Correct recombinants (and orientation of inserts) were confirmed by restriction mapping (using twelve restriction digests which cut in both vector and insert), and by reamplification of the full length insert from recombinant plasmid using the primers described above. This procedure yielded plasmid SV-PNP.

### Transfection of T84 Colon Carcinoma Cells

Cationic liposome mediated gene transfer was used to transfect T84 colon carcinoma cells. Briefly, 6µg of plasmid containing PNP or LacZ was added to 10 µg of a 1:1 molar mixture of DOTMA/DOPE (Lipofectin™ (GIBCO/BRL, Gaithersburg, MD)) in a final volume of 200 µl DMEM/F12 serum free medium. After a 10 minute incubation at room temperature, the DNA-lipid mixture was added to 500 µl serum free medium and was used to cover the cells within a well. Four hours later, transfection medium was removed from each well and 2 ml DMEM/F12 with 10% fetal bovine serum was added.

### Transfection Efficiency

The LacZ gene was transfected into T84 cells as described above. Briefly, using a lipid-mediated gene transfer protocol identical to that described above, 6 µg of plasmid containing the *E. coli* LacZ gene under the control of the SV-40 early promoter was transferred into 1-2 x 10⁵ T84 cells. 48 hours after transfection, cells were washed 3 times in PBS, fixed at 4°C x 10 minutes in 0.2% glutaraldehyde, (in 80 mM NaHPO₂), rinsed 2 times with PBS, and then stained in a solution containing 80 mM Na₂HPO₄, 20 mM NaH2PO4, 1.3 mM MgCl₂, 3 mM K₃Fe(CN)₆, 3 mM K₄Fe(CN)₆ and 1 mg/ml X-gal (5-bromo-4-chloro-3-indolyl-β-D-galactopyranoside). 12 hours after staining, 0.1 - 1% of the cells treated with β-galactosidase DNA stained positive for gene expression.

X-gal staining of these cells two days after transfection indicated an overall transfection efficiency of 0.1-1% (as determined by percentage of blue cells). No positive cells were observed in untreated T84 cells, in cells treated with lipid alone, or plasmid DNA alone. Similar conclusions were reached using a LacZ reporter gene containing a nuclear targeting sequence and leading to nuclear staining of recombinant cells.

### Addition of MeP-dR

48 hours following transfection, fresh medium was added and MeP-dR (1 mg/ml in PBS) was added directly to the cells to achieve the desired final concentrations. Cell viability was measured 5 days following treatment as described above for the MeP-dR toxicity study.

In one experiment, MeP-dR (160 µM) was added to wells containing untransfected cells, or cells transfected with 10, 20, or 40 µg (1, 2, and 3 of Figure 1, respectively) of cDMA containing either the *E. coli* PNP or LacZ genes under control of the SV-40 early promoter (in otherwise comparable vector contexts). After 5 days the cells were removed from each well and the number of dye excluding cells were determined with the aid of a hemacytometer. 30-50% toxicity due to the DOTMA-DOPE transfection protocol is acceptable for cationic liposome-mediated gene transfer to T84 *in vitro* when performed under optimal conditions. The results of this study are shown in Figure 1.

In an additional experiment, approximately 2 x 10⁵ cells per well were transfected as above using 6 µg of plasmid containing *E. coli* PNP cDNA. Two days after transfection, varying concentrations of MeP-dR (0, 2, 4, 20, 40 and 160 µM) were added to the wells, and after 5 days the dye excluding cells were counted with the aid of a hemacytometer. Concentrations of MeP-dR as low as 4 µM resulted in greater than 80% inhibition of cell growth.

An experiment was also performed in triplicate in which 2 x 10⁵ cells per well were transfected with LacZ or PNP using the protocol described for transfection. Two days after transfection, 16 µM MePdR was added to one set of the cultures transfected with PNP and one set of the cultures transfected with LacZ. The other PMP and LacZ transfected cultures did not receive drug. The results demonstrate minimal cell killing in all cultures except the PNP-transfected, MeP-dR treated culture.

In the above experiments, MeP-dR (160 µM) was minimally toxic to the cells that were not transfected. While expression of the LacZ gene had no influence on toxicity mediated by MeP-dR, MeP-dR killed virtually all of the cells transfected with the *E. coli* PNP (Figure 1). Substantial killing could also be seen with 16 µM MeP-dR after PNP transfection. These results indicate that low efficiency expression of *E. coli* PNP cONA (expression in less than 1% of tumor cells) was adequate for nearly 100% transfected cell and bystander cell killing. In addition, because diffusion of MeP into the medium covering the cells could have a substantial dilutional effect, it may be that an even lower fraction of tumor cells expressing *E. coil* PNP *in vivo* might be able to mediate tumor cell necrosis in the presence of MeP-dR.

### Activity of E. coli PNP on MeP-dR in Cell Extracts

The toxicity of MeP-dR in T84 cells expressing the *E. coli* PNP activity was measured in transfected T84 cells. Briefly, T84 cells transfected with 6 µg of plasmid containing either the *E. coli* PNP gene or the LacZ (β-galactosidase) gene as described above were collected by centrifugation 48 hours after transfection and resuspended in 3 volumes of 0.01 M potassium phosphate (pH 7.4), followed by incubation on ice for 15 minutes. The pellet was homogenized, and the sample was centrifuged at 100,000 x g for 60 minutes. PNP activity was measured in 100 µl volumes containing 50 mM potassium phosphate (pH 7.4), 100 µM of MeP-dR, and 1 mg/ml of protein from the cell extract. After incubation for 24 hours at 25°C, the reaction was stopped by boiling, the precipitated proteins were removed by centrifugation, and the reaction mixture was subjected to HPLC by injection onto a Spherisorb ODS1 (5 µm) column (Keystone Scientific Inc., State College, PA). The MeP-dR and MeP were eluted with a 30 min isocratic gradient of 50 mM ammonium dihydrogen phosphate buffer (pH 4.5)/acetonitrile (95/5; v/v) at a flow rate of 1 ml/min. MeP-dR and MeP were detected by their absorbance at 254 nm.

Approximately 24% of the MeP-dR was converted to MeP in extracts from the T84 colon carcinoma cells transfected with the *E. coli* PNP gene, whereas no conversion occurred in cell extracts from colon carcinoma cells transfected with the LacZ gene. Total PNP activity (human + *E. coli*) measured using inosine as substrate was not changed in T84 cells transfected with *E. coli* PNP. Thus, despite the relatively low level of expression of the *E. coli* PNP in the transfected cells, a sufficient amount of the MeP-dR was converted to kill all of the cells.

### Detection of MeP in Medium of T84 Cells Transfected with E. coli PNP

MeP-dR (160 µM) was added 48 hours after transfection of T84 cells with the *E. coli* PNP gene. Five days after the addition of MeP-dR, the medium was collected, and the proteins were precipitated by boiling. After centrifugation, the medium was analyzed for the appearance of MeP by reverse phase HPLC as described above.

MeP was detected only in the culture medium of T84 cells transfected with *E. coli* PNP. More than 75% of the MeP-dR was converted to MeP over a 5 day period in *E. coli* PNP transfected cells, but not in LacZ transfected cells. These results have significance, because they indicated that 1) untransfected and mock transfected colonic carcinoma cells lack an enzymatic mechanism for conversion of MeP-dR to MeP, 2) as predicted, MeP was readily released into the extracellular medium, so as to establish effective bystander killing, and 3) the extracellular concentrations of MeP generated by recombinant PNP were sufficient to fully explain the bystander killing which was observed. In addition, these results establish that SV-40 driven expression of the prokaryotic PNP in eukaryotic cells (as with the *E. coli* LacZ) leads to a highly active and functional enzyme. Because *E. coli* PNP is believed to assemble as a homohexamer in prokaryotic cells, the mechanisms of *E. coli* PNP oligomerization are likely to be compatible with eukaryotic protein synthesis.

### Toxicity to Nondividing Cells

Results from two experiments indicate that MeP is able to kill non-proliferating cells. This distinguishes MeP from most other currently used antitumor agents.

In the first experiment, CEM cells were cultured in 1% serum instead of the normal 10% serum for 48 hours. Under these conditions, the cells stop growing and the cell numbers stabilize at 1.5 to 2 times the original cell numbers. Cell growth continues when cells are returned to culture medium containing 10% serum. Addition of MeP at a final concentration of 10 µg/ml to CEM cell cultures after 48 hours of incubation with 1% serum caused a decline in cell numbers to approximately 25% of their original number, which indicated that MeP was toxic to non-proliferating cells.

In the second experiment, the effect of MeP on the incorporation of thymidine into DNA, uridine into RNA, and leucine into protein was determined. RNA and protein synthesis were affected most by treatment with MeP. Effects on DNA synthesis occurred only after effects on RNA and protein synthesis were evident. These results indicated that the inhibitory effect of MeP on either RNA or protein synthesis was responsible for its toxicity. These two functions are vital to all cells regardless of their proliferative state, which indicates that MeP should be toxic to both proliferating and non-proliferating cells.

### Example 2: Additional Useful Recombinant Vectors

A recombinant retrovirus was made by adding the bacterial PNP sequences to a plasmid retroviral vector that was subsequently passed through packaging cell lines for the production of virus. The retroviral vector, pLNSX (Miller and Rosman, BioTechniques, 7:980-991 (1989)), contains a cloning site that is just 3' to an SV40 early promoter which will direct transcription of a coding sequence inserted within the cloning site. The bacterial PNP sequence was ligated into linearized pLNSX. The ligation mixture was used to generate bacterial transformants that were identified by colony hybridization, and one clone (pLN/PNP) containing the PNP coding sequence in a 5' to 3' orientation relative to the SV40 promoter was amplified by standard techniques with cesium chloride gradient centrifugation. The plasmid was transfected by lipid-mediated gene transfer into the Ψ2 packaging cell line. The supernatant from these cells was harvested 48 hrs later, clarified by 0.45 µM filtration and applied to additional Ψ2 packaging cell line. In 24-36 hrs, the cells were enzymatically detached and plated at a density 1/5 the original density in media supplemented with G418 (1 g/L). Virus producing cells appeared as colonies 7-10 days later that were isolated with cloning rings and assessed for quantity and fidelity of recombinant virus production.

A recombinant adenovirus was made by adding the bacterial PNP sequences to a plasmid adenoviral vector that was subsequently passed through a packaging cell line for the production of virus. The adenoviral plasmid vector, pACCMV (Kolls, et al., Proc. Natl. Acad. Sci. (USA), 91:215-219 (1993)) was linearized with EcoRI and HindIII at the multiple cloning site which is operably linked to the cytomegalovirus (CMV) immediate early promoter. The bacterial PNP-encoding sequence was excised from the SV/PNP plasmid using NotI, and the fragment gel purified and ligated into the NotI site of pSL1180 (Pharmacia, Piscataway, NJ) to produce the plasmid designated pSL/PNP. The PNP-encoding sequence was excised from pSL/PNP with EcoRI and HindIII, gel purified, and ligated into the EcoRI and HindIII site of pACCMV to make the new plasmid designated pACCMV/PNP. Transfection of the pACCMV/PNP into cells conferred dose-dependent toxicity following exposure to the MeP-dR prodrug, confirming that the CMV promoter directed the production of therapeutic levels of the bacterial PNP. The pACCMV/PNP was cotransduced with the pJM17 vector into human embryonal carcinoma 193 cells that contain adenoviral E1A sequences necessary for viral replication.

### Tyrosinase Promoter Sequence-Directed Expression Plasmids

The human tyrosinase regulatory sequence was amplified by the polymerase chain reaction (PCR) from human genomic DNA. The genomic DNA was obtained from nucleated human blood cells by standard techniques. PCR primers A (GAT CGC TAG CGG GCT CTG AAG ACA ATC TCT CTC TGC (SEQ ID NO. 3)) and B (GAT CGC TAG CTC TTC CTC TAG TCC TCA CAA GGT CT (SEQ ID NO. 4)) amplified bp - 451 to +78 with the addition of NheI restriction enzyme sites at each end using the published sequence of Kikuchi et al., (Kikuchi, et al., *Biochim. Biophys.* Acta, *1009:* 283-286 (1989)). The PCR reaction used the following conditions for 30 cycles: 94°C x 1 min, 50°C x 2 min, 72°C x 3 min. The final product was clarified by phenol/chloroform extraction, digested with NheI, gel purified, and ligated into the NheI cloning site of the commercial luciferase vector, pGL2 Basic (Promega, Madison, WI) by standard techniques. Recombinants were screened by restriction mapping and a correctly oriented clone was identified (Tyr-Luc). A plasmid with the tyrosinase promoter in reverse orientation (Rev-Tyr-Luc), for use as a negative control, was also selected. A control vector (SV-Luc) containing the SV-40 virus early promoter and SV-40 enhancer region driving the expression of firefly luciferase (pGL2 Control vector, Promega, Madison, WI) was used to verify successful transfection of cells. To create a plasmid in which the tyrosinase promoter controlled PNP expression, the PNP gene was substituted for luciferase in the Tyr-Luc. This was accomplished using a XhoI/SalI digest to excise the full length PNP gene from SV-PNP, followed by insertion of this fragment into the XhoI/SalI sites remaining after a XhoI/SalI digest to remove the luciferase gene from Tyr-Luc.

The tyrosinase reporter constructs were tested in transient expression assays. The Lipofectin™ (GIBCO/BRL, Gaithersburg, MD) transfection protocol was used for all luciferase reporter gene experiments. Cells were seeded at 50% confluency in six-well plates and allowed to grow overnight. Immediately prior to transfection each well was washed three times with sterile phosphate buffered saline (PBS). A single well of a six-well plate was transfected with a ratio of 10 µg liposomes/10-20 µg of plasmid DNA, depending on the cell line. Liposome/DNA complexes were prepared according to manufacturer's instructions. The liposome/DNA complexes were mixed with serum free media (SFM) and a total volume of 700 µl was placed in a single well of a six-well plate. After incubation at 37°C for 14 to 16 hours, the transfection mixture was aspirated and 2 ml of complete media was added. The cells were harvested after 48 additional hours and luciferase activity was determined using the instructions and reagents of a commercial kit (Luciferase Assay System, Promega, Madison, WI). Luciferase reporter gene expression was assessed 48 hrs following transfection of various carcinoma cell lines (melanoma, liver, colon, prostate, myeloma, glial, HeLa) with a construct containing a promoterless luciferase vector ("Basic"); a luciferase gene linked to a human tyrosinase promoter in reverse orientation (incorrect orientation to transcribe the luciferase gene) (Rev-Tyr-Luc); a luciferase gene operably linked to the constitutive SV40 early promoter (SV-LUC); or a luciferase gene operably linked to a human tyrosinase promoter (correct orientation to transcribe the luciferase gene) (Tyr-Luc).

As shown in Figures 2a-d, the tyrosinase transcriptional promoter sequence specifically restricted expression of the luciferase reporter gene to which it was operably linked, to melanoma cells (Mel-1 and Mel-21). In contrast, the SV40 early promoter constitutively expressed the luciferase gene to which it was operably linked in all transfected carcinoma cell lines. The results demonstrate that tissue-specific promoter sequences can be used to transcriptionally target the expression of a heterologous enzyme to a specific tumor.

To eliminate possible toxicity associated with the non-hydrolyzable cationic lipid component of the Lipofectin™, an alternative liposome transfection vehicle was used in the killing experiments. A liposome vehicle consisting of a 1:1 (weight/weight) mixture of the cationic lipid DOTAP (1,2-dioleoyloxy-3-(trimethylammonium)-propane) and the neutral lipid DOPE (dioleoyl-phosphatidylethanolamine) (Avanti Polar Lipids) display transfection properties similar to Lipofectin™, but with less toxicity (data not shown). DOTAP/DOPE liposomes were prepared by mixing 0.5 mg of DOTAP and 0.5 mg of DOPE and evaporating the chloroform solvent. Following the addition of 500 µl of cyclohexane, the mixture was placed on dry ice and lyophilized. One ml of sterile water was added to the powdered lipids and the solution was vortexed every 5 minutes for 30 minutes. T84 or MEL-1 cells were seeded at 30% confluency in 24-well plates and allowed to grow overnight. Immediately prior to transfection, each well was washed three times with sterile PBS. To transfect a single well of a 24-well plate, 7.5 µg of DOTAP/DOPE (1 µg/µl) was mixed with 1.875 µg Of plasmid DNA (1 µg/µl) and incubated for 15 minutes. Following a 15 minute incubation, the liposome/DNA complexes were mixed with 266 µl of SFM and added to a single well of a 24-well plate. The plates were incubated for four hours at 37°C, and then the transfection mixture was aspirated and replaced with 500 µl of complete media. Using this protocol, no significant toxicity due to transfection was observed.

In cells that received the PNP or control plasmids, the media was changed two days after transfection and MeP-dR (6-methylpurine-deoxyriboside) added to the appropriate wells to a final concentration of 30 µg/ml. Four days later, the cells were fed by adding fresh media with MeP-dR (30 µg/ml) to the wells without removing the old media. Two days later (day 6), the cells were washed once with PBS, resuspended, and counted in a 20% solution of trypan blue reagent (Trypan Blue Stain 0.4%, Gibco-BRL, Gaithersburg, MD) using a hemacytometer.

Both T84 colon carcinoma cells and Mel-1 melanoma cells were transfected using DOTAP/DOPE liposomes with the SV-PNP construct, in which the constitutive SV40 early promoter is operably linked to the bacterial PNP gene; or the Tyr-PNP, in which the melanoma specific tyrosinase promoter is operably linked to the bacterial PNP gene; or the Tyr-Luc (see above); or not transfected with any recombinant construct ("no txf"). Only melanoma cells (Mel-1) transfected with the Tyr-PNP construct were susceptible to killing upon administration of the prodrug MeP-dR purine analog nucleoside as demonstrated by comparing Figure 3a, transfected T84 colon carcinoma cells, with Figure 3b, transfected Mel-1 melanoma cells. In contrast, when the constitutive SV40 early promoter was operably linked to the bacterial PNP gene (SV-PNP construct), both T84 colon carcinoma and Mel-1 melanoma cells transfected with the SV-PNP construct were susceptible to killing upon administration of the prodrug MeP-dR. These results demonstrate that transcriptional targeting of the expression of a purine analog nucleoside cleavage gene permits selective killing of specific tumor cells.

### Example 3: Method for Identifying Candidate Prodrugs for Bacterial PNP

The following method is useful to identify substrates (prodrugs) that are cleaved more efficiently by the bacterial PNP than by mammalian PNP. Prodrugs identified by this method can then be further assessed by animal studies for determination of toxicity, suitability for administration with various pharmaceutical carriers, and other pharmacological properties.

The method quantitatively measures the cleavage of substrates *in vitro.* The purine analog nucleosides (0.1 or 1.0 mM) were incubated in 500 µl of 100 mM HEPES, pH 7.4, 50 mM potassium phosphate, and with 100 µg/ml *E. coli* PNP or 0.1 unit/ml human PNP. The reaction mixtures were incubated at 25° C for 1 hr, and the reactions stopped by boiling each sample for 2 minutes. The cleavage of [¹⁴C]inosine by each enzyme was determined as a positive control. Each sample was analyzed by reverse phase HPLC to measure conversion from substrate to product. The nucleoside and purine analogs were eluted from a Spherisorb ODS1 (5 µm) column (Keystone Scientific, Inc., State College, PA) with a solvent containing 50 mM ammonium dihydrogen phosphate (95%) and acetonitrile (5%) at a flow rate of 1 ml/min. The substrates and products were detected by their absorbance at 254 nm, and were identified by comparing their retention times and absorption spectra with authentic samples.

By this analysis, MeP-dR, 2-F-dAdo, 1-deaza-2-amino-6-Cl-purine-riboside, 2-F-5'-deoxyadenosine, 2-Cl-2'-deoxyadenosine were all shown to be good substrates for bacterial PNP and poor substrates for the mammalian PNP, and thus are preferred candidate prodrugs which are eligible for further assessment for use in the methods and compositions described herein to treat malignancies (MeP-dR is a suitable prodrug, as noted above). Substrates 5'-amino-5'-deoxyadenosine, F-araA, and α-adenosine were moderate substrates for bacterial PNP and poor substrates for the mammalian PNP. Substrates xylosyl methylpurine, 2-Cl-2'-F-2'-deoxyadenosine, 2-F-2'-F-2'-deoxyadenosine, and 7-ribosyl-6-mercaptopurine were poor substrates for both enzymes, and therefore would not be candidate prodrugs. Similarly, substrates 7-ribosyl-hypoxanthine and thioguanosine were moderate to good substrates for both enzymes and also would not be candidate prodrugs for treating tumors using the compositions and methods described herein.

2-F-dAdo and F-araA have demonstrated antitumor activity not related to the production of fluoroadenine. Therefore, in methods described herein, the antitumor activity of these two substrates is likely to be potentiated by metabolism by the *E. coli* PNP. In addition, the metabolism and toxicity of these two agents can be prevented by incubation in the presence of 2'-deoxycytidine. Thus, by combining these substrates with 2'-deoxycytidine, antitumor activity related only to the production of fluoroadenine is possible.

**Table 2:**

| **Screening of Nucleosides as Substrates for *E. coli* PNP.** | | | | |
|---|---|---|---|---|
| | *E. coli* PNP | | Human PNP | |
| Substrate | 100 µM | 1 mM | 100 µM | 1 mM |
| **I. Nucleocides that are good substrates for *E. coil* PNP, but are at best poor substrates for human PNP.** | | | | |
| MeP-dR | 93 (87) | 29 (24) | 0 | 0 |
| | 91 (86) | 45 (21) | 0 (86) | 0 (47) |
| | | | | |
| FdAdo | 56 (69) | 14 (18) | 0 (70) | 0 (30) |
| | 60 (86) | 38 (21) | 0 (86) | 0 (47) |
| | | | | |
| 1-deaza-2-amino-6-Cl-purine-riboside | 62 (87) | 16 (23) | 0 (88) | 0 (52) |
| | 41 (86) | 15 (21) | 0 (86) | 0 (47) |
| | | | | |
| 2-F-5'-deoxy-adenosine | 81 (86) | 30 (21) | 0 (88) | 0 (50) |
| | 65 (86) | 44 (21) | 0 (86) | 0 (47) |
| | | | | |
| 2-Cl-2'-deoxy-adenosine | 41 (86) | - | 0 (87) | - |

| **II.** | **Nucleosides that are moderate substrates for *E. coli* PNP, but are at best poor substrates for human PNP.** | | | |
|---|---|---|---|---|
| 5'-amino-5'-deoxy-adenosine | 5 (86) | 1 (19) | 0 (89) | 0 (53) |
| | 9 (86) | 5 (21) | 0 (86) | 0 (47) |
| | | | | |
| F-araA | 3 (86) | 3 (21) | 0 (88) | 0 (50) |
| | 5 (86) | 12 (21) | 0 (86) | 0 (47) |
| | | | | |
| α-adenosine | 0 (86) | 0 (21) | 0 (88) | 0 (50) |
| | 3 (86) | 2 (21) | 0 (86) | 0 (47) |
| | | | | |

| **XII.** | **Nucleosides that are at best poor substrates for both enzymes.** | | | |
|---|---|---|---|---|
| xylosyl methyl-purine | 0 (86) | 0 (21) | 0 (88) | 0 (50) |
| | 0 (86) | 0 (21) | 0 (86) | 0 (47) |
| | | | | |
| 2-Cl-2'-F-2'-deoxy-adenosine | 0 (86) | 0 (21) | 0 (88) | 0 (50) |
| | 0 (86) | 0 (21) | 0 (86) | 0 (47) |
| | | | | |
| 2-F-2'-F-2'-deoxy-adenosine | 0 (86) | 0 (21) | 0 (88) | 0 (50) |
| | 0 (86) | 0 (21) | 0 (86) | 0 (47) |
| | | | | |
| 7-ribosyl-6-mercapto-purine | 0 | 0 | 0 | 0 |
| | 0 (86) | 0 (21) | 0 (86) | 0 (47) |
| | | | | |

| **IV.** | **Nucleosides that are substrates for both enzymes.** | | | |
|---|---|---|---|---|
| 7-Ribosyl-hypoxanthine | 16 (86) | 30 (21) | 3 (86) | 5 (47) |
| | 49 (86) | 38 (21) | 73 (86) | 73 (47) |
| thioguanosine | 49 (86) | 38 (21) | 73 (86) | 48 (47) |

In Table 2, above, each of the numbers represent the percent conversion of the purine analog nucleoside by the phosphorylase indicated. The numbers in parentheses are percent conversion of the inosine to hypoxanthine in the same experiment.

### Example 4: In Vivo Treatment with Bacterial PNP and MeP-dR

The utility of the bacterial PNP and prodrug such as MeP-dR to inhibit cancer growth in *vivo* was demonstrated in mice engrafted with tumors expressing the bacterial PNP gene. The first step of this study required the production of a recombinant retrovirus containing a constitutively expressed bacterial PNP gene, as described in Example 2, above. The bacterial PNP encoding sequence was excised from the SV/PNP plasmid and ligated by standard techniques into the HindIII site of pLNSX vector (Miller, et al., *BioTechnique*, 7:980-990, 1989). The resulting vector, pLN/PNP, used the SV40 early promoter to constitutively direct the bacterial PNP transcription. This plasmid vector was transfected into the Ψ2 packaging cell line, the supernatant collected from these cells 48 hrs later used to infect additional Ψ2 packaging cells. Twenty four hours after applying the supernatant, the Ψ2 cells were enzymatically detached and plated at a lower density (1:5-1:10) in media containing G418 (1 gm/L) in order to select for clones containing the retroviral sequences. Several clones were selected and titers of clones determined by standard techniques (see Rousculp, et al., *Human Gene Therapy,* 3:471-477 (1992)) for full description of these standard methods). A clone with the highest titer was selected as the source of recombinant, LN/PNP virus, and used to infect tumor cells.

The murine mammary carcinoma cell line, 16c, was modified to constitutively express the bacterial PNP by infection with the LN/PNP virus. The 16c cells were plated at a subconfluent density, and the LN/PNP virus contained within the supernatant from the Ψ2-producer line (described above) was applied in the presence of polybrene (5 µg/ml) for several hours. The media was changed to normal media for 24 hrs, after which the cells were enzymatically detached and plated at a lower density in media containing G418 (1 gm/L) to select infected cells. A polyclonal mixture of G418 resistant cells, to be referred to here as "16C-PNP cells", was amplified in number for engraftment into mice.

Athymic nude mice were engrafted with the 16c-PNP cells. Each mouse received 2 x 10⁶ cells subcutaneously (SQ) in the left flank on day 1.
The results are shown in Figure 4. Control animals (n=4) were maintained under normal nude mouse conditions that resulted in measurable tumors by day 13. The tumors in all of the control mice continued to increase in size through day 22 following engraftment. The early treatment group (n=4) was treated by intraperitoneal (IP) injections of 6-MePdR at 100 mg/kg, a dose just below the maximum tolerated dose, each day for the first 4 days (days 1-4). One of these mice was sacrificed at day 8 to study tumor histology, and two more died at day 20, from undetermined causes, possibly due to the very high levels of prodrug administered. Importantly, none of the mice had any detectable tumor up to 18 days post-engraftment. One mouse developed a very small tumor at day 22. The late treatment group (n=4) was treated by intraperitoneal (IP) injections of 6-MePdR at 100 mg/kg each day on days 13, 14, and 15 post engraftment. All of the late treatment group had tumors of comparable size to the controls on day 13. Unlike the controls, the tumors in the late treatment group did not increase in size after day 15. All of these animals survived for the complete experiment. These results clearly show that the combination of the bacterial PNP plus prodrug causes a reduction in tumor growth *in vivo*.

Throughout this application various publications are referenced by citation. The disclosures of these publications in their entireties are hereby incorporated by reference into this application in order to more fully describe the state of the art to which this invention pertains.

## Claims

1. A composition for killing targeted mammalian cells comprising:
a) an enzyme that cleaves a purine analog substrate to yield a cytotoxic purine analog, and
b) an effective amount of the purine analog substrate to kill the targeted cells when cleaved by the enzyme.

2. The composition according to claim 1, wherein the targeted cells are tumor cells.

3. The composition according to claim 1, wherein the targeted cells are virally infected cells.

4. The composition according to claim 1, wherein the targeted cells are engineered to produce a therapeutic protein in addition to the enzyme that cleaves a purine analog substrate.

5. The composition according to claim 1, wherein the enzyme is a bacterial PNP or hydrolase.

6. The composition according to claim 1, wherein the enzyme is a modified mammalian PNP or hydrolase.

7. The composition according to claim 1, wherein the enzyme is targeted to the cells.

8. The composition according to claim 7, wherein the enzyme is conjugated to an antibody.

9. The composition according to claim 1, wherein the enzyme is encoded by a gene provided to the cells.

10. The composition according to claim 9, wherein the gene encoding the enzyme is operably linked to a tissue-specific promoter or a constitutive promoter.

11. The composition according to claim 10, wherein the gene encodes E. coli PNP.

12. The composition according to claim 11, wherein the gene encoding E. coli PNP is operably linked to a tyrosinase gene promoter.

13. The composition according to claim 11, wherein the gene encoding E. coli PNP is operably linked to a promoter selected from the group consisting of SV40 early promoter, Moloney murine sarcoma virus long terminal repeat, mouse mammary tumor virus long terminal repeat, and the cytomegalovirus early promoter.

14. The composition according to claim 9, wherein the gene is provided in a vector.

15. The composition according to claim 9, wherein the gene is provided in a carrier molecule such as polymeric films, gels, microparticles, and liposomes.

16. The composition according to claim 1, wherein the purine analog substrate is selected from the group consisting of 9-(β-D-2-deoxyerythropentofuranosyl)-6-methylpurine, 2-amino-6-chloro-1-deazapurine riboside, 7-ribosyl-3-deazaguanine, arabinofuranosyl 2-fluoroadenine,2-fluoro-2'-deoxyadenosine, 2-fluoro-5'-deoxyadenosine, 2-chloro-2'-deoxy-adenosine,5'-amino-5'-deoxy-adenosine,α-adenosine.

17. A method of making a composition for killing targeted mammalian cells comprising:
(a) isolating an enzyme or the gene encoding the enzyme that cleaves a purine analog substrate to yield a cytotoxic purine analog, and
(b) selecting an effective amount of the purine analog substrate to kill the targeted cells when the purine analog substrate is cleaved by the enzyme.

18. The method according to claim 17, wherein the targeted cells are tumor cells.

19. The method according to claim 17, wherein the targeted cells are virally infected cells.

20. The method according to claim 17, wherein the targeted cells are engineered to produce a therapeutic protein in addition to the enzyme that cleaves a purine analog substrate.

21. The method according to claim 17, wherein the isolated enzyme is a modified mammalian PNP or hydrolase.

22. The method according to claim 17, wherein the enzyme or a gene encoding the enzyme is isolated from bacteria, fungi, or human parasite species.

23. The method according to claim 17, wherein the enzyme or gene encoding the enzyme is isolated from E. coli.

24. The method according to claim 23 further comprising the step of operably linking the gene encoding the enzyme to a tissue-specific promoter or to a constitutive promoter.

25. The method according to claim 24, wherein the gene encodes E. coli PNP which is linked operably to a tyrosinase gene promoter.

26. The method according to claim 24, wherein the gene encodes E. coli PNP which is operably linked to a promoter selected from the group consisting of SV40 early promoter, Moloney murine sarcoma virus long terminal repeat, mouse mammary tumor virus long terminal repeat, and the cytomegalovirus early promoter.

27. The method according to claim 17, wherein the purine analog substrate is selected from the group consisting of 9-(β-D-2-deoxyerythropentofuranosyl)-6-methylpurine, 2-amino-6-chloro-1-deazapurine riboside, 7-ribosyl-3-deazaguanine, arabinofuranosyl 2-fluoroadenine, 2-fluoro-2'-deoxyadenosine, 2-fluoro-5'-deoxyadenosine, 2-chloro-2'-deoxy-adenosine, and 5'-amino-5'-deoxy-adenosine, α-adenosine.

28. A method for screening for a prodrug useful in a method of killing targeted mammalian cells comprising comparing the rate of cleavage of a compound to produce a purine analog by a mammalian PNP with the rate of cleavage by a non-mammalian PNP, a modified mammalian PNP, or equivalent cleavage enzyme.

## Patentansprüche

1. Zusammensetzung zum Töten von Ziel-Säugerzellen, umfassend:
a) ein Enzym, das ein Purin-analoges Substrat zu einem zytotoxischen Purinanalogen spaltet und
b) eine wirksame Menge des Purin-analogen Substrats zum Töten der Zielzellen, wenn es durch das Enzym gespalten wird.

2. Zusammensetzung nach Anspruch 1, wobei die Zielzellen Tumorzellen sind.

3. Zusammensetzung nach Anspruch 1, wobei die Zielzellen viral infizierte Zellen sind.

4. Zusammensetzung nach Anspruch 1, wobei die Zielzellen aufgebaut sind, um ein therapeutisches Protein, zusätzlich zu dem Enzym, das ein Purin-analoges Substrat spaltet, zu erzeugen.

5. Zusammensetzung nach Anspruch 1, wobei das Enzym eine bakterielle PNP oder Hydrolase ist.

6. Zusammensetzung nach Anspruch 1, wobei das Enzym eine modifizierte Säuger-PNP oder Hydrolase ist.

7. Zusammensetzung nach Anspruch 1, wobei das Enzym auf die Zellen gerichtet ist.

8. Zusammensetzung nach Anspruch 7, wobei das Enzym an einen Antikörper konjugiert ist.

9. Zusammensetzung nach Anspruch 1, wobei das Enzym durch ein für die Zellen bereitgestelltes Gen codiert ist.

10. Zusammensetzung nach Anspruch 9, wobei das Gen, das das Enzym codiert, an einen Gewebs-spezifischen Promotor oder einen konstitutiven Promotor wirkungsfähig gebunden ist.

11. Zusammensetzung nach Anspruch 10, wobei das Gen E. coli PNP codiert.

12. Zusammensetzung nach Anspruch 11, wobei das Gen, das E. coli PNP codiert, wirkungsfähig an einen Tyrosinase-Genpromotor gebunden ist.

13. Zusammensetzung nach Anspruch 11, wobei das Gen, das E. coli PNP codiert, wirkungsfähig an einen Promotor, ausgewählt aus der Gruppe, bestehend aus SV40-frühem Promotor, einer langen terminalen Wiederholung von Moloney-murinem Sarcomvirus, einer langen terminalen Wiederholung von Maus-Brustdrüsen-Tumorvirus und dem Zytomegalovirus-frühen Promotor, gebunden ist.

14. Zusammensetzung nach Anspruch 9, wobei das Gen in einem Vektor bereitgestellt wird.

15. Zusammensetzung nach Anspruch 9, wobei das Gen in einem Trägermolekül, wie Polymerfolien, Gele, Mikropartikel und Liposomen, bereitgestellt wird.

16. Zusammensetzung nach Anspruch 1, wobei das Purin-analoge Substrat ausgewählt ist aus der Gruppe, bestehend aus 9-(β-D-2-Desoxyerythropentofuranosyl)-6-methylpurin, 2-Amino-6-chlor-1-desazapurinribosid, 7-Ribosyl-3-desazaguanin, Arabinofuranosyl-2-fluoradenin, 2-Fluor-2'-desoxyadenosin, 2-Fluor-5'-desoxyadenosin, 2-Chlor-2'-desoxyadenosin, 5'-Amino-5'-desoxyadenosin, α-Adenosin.

17. Verfahren zur Herstellung einer Zusammensetzung zum Töten von Ziel-Säugerzellen, umfassend:
a) Isolieren eines Enzyms oder des Gens, das das Enzym codiert, das ein Purin-analoges Substrat spaltet, unter Gewinnung eines zytotoxischen Purinanalogen und
b) Auswählen einer wirksamen Menge des Purin-analogen Substrats zum Töten der Zielzellen, wenn das Purin-analoge Substrat durch das Enzym gespalten wird.

18. Verfahren nach Anspruch 17, wobei die Zielzellen Tumorzellen sind.

19. Verfahren nach Anspruch 17, wobei die Zielzellen viral infizierte Zellen sind.

20. Verfahren nach Anspruch 17, wobei die Zielzellen aufgebaut sind, um ein therapeutisches Protein, zusätzlich zu dem Enzym, das ein Purin-analoges Substrat spaltet, zu erzeugen.

21. Verfahren nach Anspruch 17, wobei das isolierte Enzym eine modifizierte Säuger-PNP oder Hydrolase darstellt.

22. Verfahren nach Anspruch 17, wobei das Enzym oder ein Gen, das das Enzym codiert, aus Bakterien, Pilzen oder Human-Parasiten-Arten isoliert wird.

23. Verfahren nach Anspruch 17, wobei das Enzym oder das Gen, das das Enzym codiert, aus E. coli isoliert wird.

24. Verfahren nach Anspruch 23, weiterhin umfassend den Schritt von wirkungsfähigem Binden des das Enzym codierenden Gens an einen gewebsspezifischen Promotor oder an einen konstitutiven Promotor.

25. Verfahren nach Anspruch 24, wobei das Gen, das E. coli PNP codiert, wirkungsfähig an einen Tyrosinase-Genpromotor gebunden ist.

26. Verfahren nach Anspruch 24, wobei das Gen, das E. coli PNP codiert, wirkungsfähig an einen Promotor, ausgewählt aus der Gruppe, bestehend aus SV40-frühem Promotor, einer langen terminalen Wiederholung von Moloney-murinem Sarcomvirus, einer langen terminalen Wiederholung von Maus-Brustdrüsen-Tumorvirus und dem Zytomegalovirusfrühen Promotor, gebunden ist.

27. Verfahren nach Anspruch 17, wobei das Purin-analoge Substrat ausgewählt ist aus der Gruppe, bestehend aus 9-(β-D-2-Desoxyerythropentofuranosyl)-6-methylpurin, 2-Amino-6-chlor-1-desazapurinribosid, 7-Ribosyl-3-desazaguanin, Arabinofuranosyl-2-fluoradenin, 2-Fluor-2'-desoxyadenosin, 2-Fluor-5'-desoxyadenosin, 2-Chlor-2'-desoxyadenosin und 5'-Amino-5'-desoxyadenosin, α-Adenosin.

28. Verfahren zum Screening eines Prodrugs, das in einem Verfahren zum Töten von Ziel-Säugerzellen verwendbar ist, umfassend Vergleichen der Spaltungsgeschwindigkeit einer Verbindung zur Erzeugung eines Purinanalogen durch eine Säuger-PNP, mit der Spaltungsgeschwindigkeit durch eine Nicht-Säuger-PNP, eine modifizierte Säuger-PNP oder ein äquivalentes Spaltungsenzym.

## Revendications

1. Composition pour tuer des cellules de mammifère ciblées comprenant :
a) une enzyme qui clive un substrat d'analogue de purine pour donner un analogue de purine cytotoxique, et
b) une quantité efficace de substrat d'analogue de purine pour tuer les cellules ciblées lorsqu'elles sont clivées par l'enzyme.

2. Composition selon la revendication 1, dans laquelle les cellules ciblées sont des cellules tumorales.

3. Composition selon la revendication 1, dans laquelle les cellules ciblées sont des cellules infectées viralement.

4. Composition selon la revendication 1, dans laquelle les cellules ciblées sont traitées pour produire une protéine thérapeutique en plus de l'enzyme qui clive un substrat d'analogue de purine.

5. Composition selon la revendication 1, dans laquelle l'enzyme est une PNP bactérienne ou une hydrolase.

6. Composition selon la revendication 1, dans laquelle l'enzyme est une PNP de mammifère modifiée ou une hydrolase.

7. Composition selon la revendication 1, dans laquelle l'enzyme est ciblée vers les cellules.

8. Composition selon la revendication 7, dans laquelle l'enzyme est conjuguée à un anticorps.

9. Composition selon la revendication 1, dans laquelle l'enzyme est codée par un gène fourni aux cellules.

10. Composition selon la revendication 9, dans laquelle le gène codant l'enzyme est fixé de manière fonctionnelle à un promoteur spécifique à un tissu ou à un promoteur constitutif.

11. Composition selon la revendication 10, dans laquelle le gène code E.coli PNP.

12. Composition selon la revendication 11, dans laquelle le gène codant E. coli PNP est fixé de manière opérationnelle à un promoteur du gène de la tyrosinase.

13. Composition selon la revendication 11, dans laquelle le gène codant E.coli PNP est fixé de manière fonctionnelle à un promoteur choisi dans le groupe comprenant le promoteur précoce SV40, la répétition terminale longue du virus du sarcome murin Moloney, la répétition terminale longue du virus du cancer mammaire de la souris et le promoteur précoce du cytomégalovirus.

14. Composition selon la revendication 9, dans laquelle le gène est fourni dans un vecteur.

15. Composition selon la revendication 9, dans laquelle le gène est fourni dans une molécule véhicule comme des films polymères, des gels, des microparticules et des liposomes.

16. Composition selon la revendication 1, dans laquelle le substrat d'analogue de purine est choisi dans le groupe comprenant la 9-(β-D-2-désoxyérythropentofuranosyl)-6-méthylpurine, la 2-amino-6-chloro-1-déazapurine-riboside, la 7-ribosyl-3-déazaguanine, l'arabinofuranosyl-2-fluoroadénine, la 2-fluoro-2'-désoxyadénosine, la 2-fluoro-5'-désoxyadénosine, la 2-chloro-2'-désoxy-adénosine, la 5'-amino-5'-désoxy-adénosine, l'α-adénosine.

17. Procédé de préparation d'une composition pour tuer des cellules de mammifère ciblées comprenant :
(a) l'isolement d'une enzyme ou du gène codant l'enzyme qui clive un substrat d'analogue de purine pour donner un analogue de purine cytotoxique, et
(b) le choix d'une quantité efficace du substrat d'analogue de purine pour tuer les cellules ciblées lorsque le substrat d'analogue de purine est clivé par l'enzyme

18. Procédé selon la revendication 17, dans lequel les cellules ciblées sont des cellules tumorales.

19. Procédé selon la revendication 17, dans lequel les cellules ciblées sont des cellules infectées viralement.

20. Procédé selon la revendication 17, dans lequel les cellules ciblées sont traitées pour produire une protéine thérapeutique en plus de l'enzyme qui clive un substrat d'analogue de purine.

21. Procédé selon la revendication 17, dans lequel l'enzyme isolée est une PNP de mammifère modifiée ou une hydrolase.

22. Procédé selon la revendication 17, dans lequel l'enzyme ou un gène codant l'enzyme est isolé(e) à partir de bactéries, champignons ou d'espèces parasitaires humaines.

23. Procédé selon la revendication 17, dans lequel l'enzyme ou le gène codant l'enzyme est isolé(e) d'E. coli.

24. Procédé selon la revendication 23, comprenant en outre l'étape consistant à fixer de manière fonctionnelle le gène codant l'enzyme à un promoteur spécifique à un tissu ou à un promoteur constitutif.

25. Procédé selon la revendication 24, dans lequel le gène code E. coli PNP qui est fixé de manière opérationnelle à un promoteur du gène de la tyrosinase.

26. Procédé selon la revendication 24, dans lequel le gène code E. coli PNP qui est fixé de manière opérationnelle à un promoteur choisi dans le groupe comprenant le promoteur précoce SV40, la répétition terminale longue du virus du sarcome murin Moloney, la répétition terminale longue du virus du cancer mammaire de la souris et le promoteur précoce du cytomégalovirus.

27. Procédé selon la revendication 17, dans lequel le substrat d'analogue de purine est choisi dans le groupe comprenant la 9-(β-D-2-désoxyérythropentofuranosyl)-6-méthylpurine, la 2-amino-6-chloro-1-déazapurine-riboside, la 7-ribosyl-3-déazaguanine, l'arabinofuranosyl-2-fluoroadénine, la 2-fluoro-2'-désoxyadénosine, la 2-fluoro-5'-désoxyadénosine, la 2-chloro-2'-désoxyadénosine, la 5'-amino-5'-désoxy-adénosine, l'α-adénosine.

28. Procédé pour sélectionner un promédicament utile dans un procédé pour tuer des cellules de mammifère ciblées comprenant la comparaison du taux de clivage d'un composé pour produire un analogue de purine par une PNP de mammifère avec le taux de clivage par une PNP non issue d'un mammifère, une PNP de mammifère modifiée ou une enzyme de clivage équivalente.
